# EUROPEAN PATENT SPECIFICATION

(11) **EP 1 274 729 B1**
(45) Date of publication and mention of the grant of the patent: **02.04.2008**
(21) Application number: 01923707.2
(22) Date of filing: 02.04.2001
(51) Int. Cl.: C07K 14/705

(54) **G PROTEIN COUPLED RECEPTOR**
G-PROTEIN GEKOPPELTER REZEPTOR
RECEPTEUR COUPLE AUX PROTEINES G

(30) Priority: 04.04.2000 GB 0008252
(43) Date of publication of application: 15.01.2003
(73) Proprietor: Janssen Pharmaceutica NV, 2340 Beerse (BE)
(72) Inventor: BENDER, Eckhard, B-2340 Beerse (BE); BUIST, Arjan, B-2340 Beerse (BE); ERCKEN, Martine, B-2340 Beerse (BE); BAGGERMAN, Geert Luc Elisabeth, B-3000 Leuven (BE); JURZAK, Mirek, 2340 Beerse (BE); SCHOOFS, Liliane, Amelie, Henrica, 3000 Leuven (BE); LUYTEN, Walter, Herman, Maria, Louis, 2340 Beerse (BE)
(74) Representative: Bird, Ariane
(86) International application number: PCT/EP2001/003688
(87) International publication number: WO 2001/074902

(56) References cited:
- DATABASE AAGENESEQ [Online] ACCESSION NUMBER AAY30162, 26 November 1999 (1999-11-26) AHMAD S ET AL.: "HUMAN DORSAL ROOT RECEPTOR 4 HDRR4" XP002172188 & WO 99 32519 A (ASTRA PHARMA INC.) 1 July 1999 (1999-07-01)
- DATABASE EMBL [Online] ACCESSION NUMBER AZ346449, 3 October 2000 (2000-10-03) DUNN D ET AL.: "MOUSE WHOLE GENOME SCAFFOLDING WITH PAIRED END READS FROM 10KB PLASMID INSERTS" XP002172212
- DATABASE EMBL [Online] ACCESSION NUMBER AC027026, 27 March 2000 (2000-03-27) BIRREN B. ET AL.: "HOMO SAPIENS CHROMOSOME 11, CLONE RP11-589F4" XP002172213
- DATABASE EMBL [Online] ACCESSION NUMBER AC023078, 14 February 2000 (2000-02-14) BIRREN B ET AL.: "HOMO SAPIENS CHROMOSOME 11, CLONE RP11-583F24" XP002172214

## Description

### Field of the Invention

The present invention relates to a novel mammalian G protein coupled receptor, to nucleic acid encoding it and to its use in assays.

### Background to the Invention

GTP-binding proteins (G proteins) act as intermediaries between binding of ligands such as hormones and other chemical mediators to G protein coupled receptors (GPCRs) and activation of intracellular effectors. Upon binding of a ligand to a GPCR, the cytoplasmic domains of the receptor undergo conformational changes which enable interaction of the receptor with a G protein, which in turn enables activation of intracellular intermediaries such as adenylate cyclase, phospholipase C or ion channels. Such a system allows amplification of the original signal as many second messengers can be produced in response to the binding of a single ligand at the GPCR. Through this mechanism, cells are able to sense and respond to alterations in their external environment.

G protein coupled receptors form a superfamily of integral plasma membrane proteins, each receptor sharing the common feature of seven hydrophobic transmembrane domains, each of which is 20 -30 amino acids long and which are linked by hydrophilic amino acid sequences of varied length. The amino terminus of the receptor is extracellular with the carboxy terminus found in the cytoplasm of the cell.

GPCRs are found in a wide range of tissues and cell types and are involved in many different physiological processes. They are activated by a broad range of ligands, for example, hormones such as luteinizing hormone, follicle stimulating hormone, chorionic gonadotrophin, thyrotropin, adrenocorticotrophin, glucagon and vasopressin; neurotransmitters such as 5-HT, acetylcholine (muscarinic AchR), histamine, prostaglandins, calcitonin, leukotrienes and Ca²⁺. The broad distribution and wide variety of roles of GPCRs indicate that GPCRs may play important roles in a variety of pathological conditions. Indeed, GPCRs have been found to be involved in diseases related to bronchoconstriction, hypertension, inflammation, hormonal disturbance, diabetes, apoptosis, nociception, facilitation of neurotransmission and tremor disorders.

One group of GPCRs of particular interest are those which act as receptors for nucleotides. Nucleotide receptors are structured into three families: P1, P2Y and P2X (Ralevic and Burnstock, 1998 Pharmacological Reviews 50(3):413-492), of which the P1 and P2Y families are GPCRs. P1 receptors are activated by adenosine and do not bind adenine (Bruns et al., Proc. Natl. Acad. Sci., USA, 77(50: 5547-51, 1980; Schwabe and Trost, 1980 Naunyn Schmiedebergs Arch Pharmacol. 313(3):197-87; Schwabe et al., 1982, Naunyn Schmiedebergs Arch Pharmacol. 321(1):84-87). Members of the P1 receptor family are divided into three subfamilies, known as the adenosine type 1 family, the adenosine type 2 family and the adenosine type 3 family.

The P2Y family contains four families of GPCRs activated by nucleotides. In detail, these are P2Y1 (activated by ADP and ATP), P2Y2 (also called P2U, these GPCRs are activated at equal potency by ATP and UTP), P2Y3 and P2Y6 (relative agonist strength is UDP>UTP>ADP) and P2Y4 (UTP>ATP). A number of other GPCRs having sequence homology to the P2Y2 group were initially thought to be nucleotide receptors as well (P2Y5, P2Y7, P2Y9 and P2Y10), but are now known not to be activated by nucleotides.

The P2X family is a group of unrelated proteins that is not linked to G proteins. Members of this group are ligand gated ion channels.

Because G protein coupled receptors play such an important role in a wide variety of cellular and physiological processes, there is ongoing interest in assessing the function of such receptors and their potential roles in a variety of diseases.

### Summary of the Invention

In the course of research into GPCRs, the present inventors have discovered a novel GPCR, which represents a new class of GPCR and which has been termed adenine binding GPCR. When the sequence of the novel GPCR in the rat was compared with those available in the prior art, the inventors found to their surprise that the closest related GPCR shares only approximately 50% sequence identity with the novel receptor, suggesting that the novel GPCR represents a member of a new class of GPCR. Further studies identified the natural ligand of the receptor to be adenine. This is the first demonstration of a GPCR which has adenine as its natural ligand, suggesting that the GPCR represents a new class of GPCR. Indeed, this is the first demonstration of a receptor of any kind which has adenine as its natural receptor and, as the novel receptor has been found to be present in the mammalian cortex, represents the first disclosure of adenine as a neurotransmitter. Further studies identified six human homologues of the receptor.

The present invention discloses an adenine binding G protein coupled receptor. In particular, the present invention discloses an isolated polypeptide having the sequence of SEQ ID NO:2 or SEQ ID NO:4. Polypeptides which are fragments of said polypeptide of SEQ ID NO:2 or SEQ ID NO:4 are also disclosed, said fragments being of at least 10, for example at least 20, 30 40, 50, 75, 100 or 150 or more amino acids in size. Such fragments may be derived from the N-terminal region of SEQ ID NO:2 or SEQ ID NO:4 respectively. Fragments including the N-terminal region may be used to reconstitute the extracellular portion of the receptor to provide receptor binding sites. Preferably, fragments will retain the ability to bind adenine.

In another embodiment, the invention discloses an isolated polypeptide having at least 50% and preferably at least 60%, 70%, 80%, 90%, 95% or 98% sequence identity to the polypeptide of SEQ ID NO:2 or SEQ ID NO:4. Such polypeptides desirably retain the ability to bind adenine and preferably to form a receptor that can be activated by adenine. Similarly, polypeptides which are fragments of at least 20 amino acids of these polypeptides preferably having at least 50% and preferably at least 60%, 70%, 80%, 90%, 95% or 98% identity to a fragment of SEQ ID NO:2 or SEQ ID NO:4 of corresponding length, including N-terminal fragments, are disclosed. Such polypeptides desirably retain the ability to bind adenine and preferably to form a receptor that can be activated by adenine.

The present invention also discloses an isolated nucleic acid sequence encoding the polypeptide of SEQ ID NO:2 or SEQ ID NO:4. In a preferred aspect, the isolated sequence is that of SEQ ID NO:1 or SEQ ID NO:3. The invention also discloses a DNA molecule encoding the polypeptide of SEQ ID NO:2 or SEQ ID NO:4. Also disclosed by the invention are isolated nucleic acids encoding the polypeptides mentioned in the previous two paragraphs. Further disclosed are DNA molecules encoding said polypeptides.

Nucleic acids which are disclosed further include nucleic acids which comprise a sequence having at least 50% and preferably at least 60%, 70%, 80%, 90%, 95% or 98% sequence identity to the nucleic acid sequences of SEQ ID NO: 1, SEQ ID NO: 3, SEQ ID NO:5, 6,7,8 or 9 or their complements. Preferably, these sequences will hybridise to the corresponding nucleic acid under conditions controlled to minimise non-specific binding. Preferably stringent to moderately stringent hybridisation conditions are preferred. Suitable conditions include, e.g. for detection of sequences that are about 80-90% identical, hybridization overnight at 42°C in 0.25M Na₂HPO₄, pH 7.2, 6.5% SDS, 10% dextran sulfate and a final wash at 55°C in 0.1X SSC, 0.1% SDS. For detection of sequences that are greater than about 90% identical, suitable conditions include hybridization overnight at 45°C in 0.25M Na₂HPO₄, pH 7.2, 6.5% SDS, 10% dextran sulfate and a final wash at 60°C in 0.1X SSC, 0.1% SDS.

It will be appreciated that such nucleic acids do not necessarily encode "full length" polypeptides, and will thus include nucleic acids which represent, for example, mutant forms of the adenine binding GPCR gene in which the coding sequence has been prematurely terminated by either a substitution resulting in a stop codon or a frameshift mutation.

The invention also discloses nucleic acids which are fragments of the nucleic acids encoding a polypeptide of the invention. In one aspect, the invention discloses nucleic acids primers which consist essentially of from 15 to 50, for example from 15 to 35, 18 to 35, 15 to 24, 18 to 30, 18 to 21 or 21 to 24 nucleotides of a sequence encoding a polypeptide of the invention or its complement.

Nucleic acids and polypeptides of the invention may be used therapeutically to treat disease. In particular, they may be used to treat diseases, the pathology of which is associated with action at purine receptors, particularly those associated with mutation or downregulation of expression of native adenine binding GPCRs. In specific, they may be used as anticonvulsives, sedatives, hypnotics, hypotensives, antiarrhytmics, negative chronotropics, dromotropics and inotropics, or in the treatment of pathological tremor disorders, ataxia, or of disease associated with nociception or the facilitation of neurotransmission. Furthermore, as well as finding use in disorders associated with the CNS, they may be used as inhibitors of platelet aggregation, stimulators of NO production by vascular endothelial cells in the cardiovasculor system, as inhibitors of gastric secretion or in disease associated with apoptosis, bronchoconstriction, vasodilation or inflammation.

In a further aspect, there are disclosed vectors comprising the sequences of said nucleic acids, particularly expression vectors comprising a promoter operably linked to the nucleic acid sequences of the invention. The vectors may be carried by a host cell, and expressed within said cell. Following said expression, polypeptides of the invention may be recovered.

In a further aspect, the invention discloses assay methods for the identification of substances which bind to or modulate the activity of polypeptides of the invention. In particular it is envisaged that such substances which may be peptide or non-peptide be used in methods of treatment as described above.

As the present inventors are the first to identify a receptor of any kind which has adenine as its natural ligand and, as the novel receptor has been found to be present in the mammalian cortex, the inventors are the first to disclose adenine as a neurotransmitter. Therefore, the present invention opens up the possibility of using adenine itself and/or compounds mimicking or antagonising adenine in therapeutic applications. Therefore the invention further discloses adenine for use in a method of treatment of a human or animal body. In particular, it is envisaged that adenine be used for treatment of disease, the pathology of which is associated with action at purine receptors, particularly adenine receptors. Furthermore, the invention discloses the use of adenine in the preparation of a medicament for the treatment of any of the diseases or conditions described above.

The invention also discloses antibodies and binding fragments thereof capable of selectively binding to polypeptides of the invention. The antibodies and binding fragments may therefore be used in diagnostic tests for the identification of the polypeptides of the invention in biological samples.

These and other aspects of the invention are described herein in more detail.

### Description of sequences.

SEQ ID NO:1 is the nucleotide sequence for rat adenine binding GPCR.
SEQ ID NO:2 is the amino acid sequence for rat adenine binding GPCR.
SEQ ID NO:3 is the nucleotide sequence for human adenine binding GPCR1.
SEQ ID NO:4 is the amino acid sequence for human adenine binding GPCR1.
SEQ ID NO:5 is the nucleotide sequence for human adenine binding GPCR2.
SEQ ID NO:6 is the nucleotide sequence for human adenine binding GPCR3.
SEQ ID NO:7 is the nucleotide sequence for human adenine binding GPCR4.
SEQ ID NO:8 is the nucleotide sequence for human adenine binding GPCR5.
SEQ ID NO:9 is the nucleotide sequence for human adenine binding GPCR6.

### Brief Description of the Drawings

**Figure 1** illustrates sequence alignment of the amino acid sequences of the rat adenine binding GPCR (SEQ ID NO:2) and human adenine binding GPCR1 (SEQ ID NO:4)
**Figure 2** illustrates activation of rat adenine binding GPCR by porcine cortex extract C18 fractions after transient transfection into HEK293 cells expressing the Gαqo chimera.
**Figure 3** illustrates activation of rat adenine binding GPCR by porcine cortex extract C18 fractions after transient transfection into HEK293 cells expressing the Gαqi chimera.
**Figure 4** illustrates the stimulation of Ca²⁺ transients in HEK293/Gαqi cells transfected with rat adenine binding GPCR/pcDNA3 by application of 3.3nM to 33µM adenine.
**Figure 5** illustrates Ca²⁺ transients elicited in HEK293/Gαqi cells transfected with rat adenine binding GPCR/pcDNA3 by application of 3.3µM uridine, guanine, hypoxanthine, xanthine, caffeine, UDP, and adenine.
**Figure 6** illustrates Ca²⁺ transients elicited in HEK293/Gαqi cells transfected with rat adenine binding GPCR/pcDNA3 by application of 3.3• M adenosine, AMP, ADP, ATP and adenine.
**Figure 7** illustrates Ca²⁺ transients elicited in HEK293/Gαqi cells transfected with rat adenine binding GPCR/pcDNA3 by application of 3• M PBS, xanthine, guanine and hypoxanthine.
**Figure 8a** illustrates stimulation of [³⁵S]GTPγS binding on CHO membranes, transiently transfected with the rat adenine binding GPCR. Data are presented as the mean of 3 independent experiments (±SEM; n=3).
**Figure 8b** illustrates stimulation of [³⁵S]GTPγS binding on wild type CHO membranes. Data are presented as the mean of 3 independent experiments (±SEM; n=3).
**Figure 9a** illustrates inhibition of forskolin stimulated cAMP formation on CHO cells, transiently transfected with the rat adenine binding GPCR. The assay was performed in 0.5% hiFCS, using the direct RIA kit from NEN. Representative curves.
**Figure 9b** illustrates inhibition of forskolin stimulated cAMP formation on CHO cells, transiently transfected with the rat adenine binding GPCR. The assay was performed in 2% hiFCS, using the direct RIA kit from NEN. Representative curves.
**Figure 10** illustrates adenine dose response curve for the stimulation of [³⁵S]GTPγS binding on CHO membranes, permanently transfected with the rat adenine binding GPCR.
**Figure 11a** illustrates inhibition of forskolin stimulated cAMP formation on CHO cells, permanently transfected with the rat adenine binding GPCR (clone 28). The figure shows representative curves obtained using 0.5 and 10% serum concentrations, each in the presence and absence of Na-butyrate induction.
**Figure 11b** illustrates inhibition of forskolin stimulated cAMP formation on wild type CHO cells. The figure shows representative curves obtained using 0.5 and 10% serum concentrations, each in the presence and absence of Na-butyrate induction.
**Figure 12a** illustrates adenine dose response curves of intracellular Ca²⁺ measurements on CHO cells, permanently transfected with the rat adenine binding GPCR (clone 28) in the presence and absence of Na-byturate.
**Figure 12b** illustrates adenine dose response curves of intracellular Ca²⁺ measurements on wild type CHO cells, in the presence and absence of Nabutyarte.

### Detailed Description of the Invention

### Nucleic acid

Nucleic acid includes DNA (including both genomic and cDNA)and RNA. Where nucleic acid according to the invention includes RNA, reference to the sequences shown in the accompanying listings should be construed as reference to the RNA equivalent, with U substituted for T.

Nucleic acid of the invention may be single or double stranded. Single stranded nucleic acids of the invention include anti-sense nucleic acids. Thus it will be understood that reference to SEQ ID NO:1 or sequences comprising SEQ ID NO:1 or fragments thereof include complementary sequences unless the context is clearly to the contrary. The same applies to SEQ ID NOs:3, 5, 6, 7, 8 and 9.

Generally, nucleic acid according to the present invention is provided as an isolate, in isolated and/or purified form, or free or substantially free of material with which it is naturally associated, such as free or substantially free of nucleic acid flanking the gene in the human genome, except possibly one or more regulatory sequence(s) for expression. Nucleic acid may be wholly or partially synthetic and may include genomic DNA, cDNA or RNA.

The invention also discloses nucleic acids which are fragments of the nucleic acids encoding a polypeptide of the invention. In one aspect, the invention discloses nucleic acids primers which consist essentially of from 15 to 50, for example from 15 to 35, 18 to 35, 15 to 24, 18 to 30, 18 to 21 or 21 to 24 nucleotides of a sequence encoding a polypeptide of the invention or its complement.

The term "consist essentially of" refers to nucleic acids which do not include any additional 5' or 3' nucleic acid sequences. In a further aspect of the invention, nucleic acids of the invention which consist essentially of from 15 to 30 nucleotides as defined above may however be linked at the 3' but preferably 5' end to short (e.g from 4 to 15, such as from 4 to 10 nucleotides) additional sequences to which they are not naturally linked. Such additional sequences are preferably linkers which comprise a restriction enzyme recognition site to facilitate cloning when the nucleic acid of the invention is used for example as a PCR primer.

Primers of the invention are desirably capable of selectively hybridising to nucleic acids encoding the polypeptides of the invention. By "selective", it is meant selective with respect to sequences encoding other purine receptors and in particular with respect to receptors other than adenine receptors. The ability of the sequence to hybridise selectively may be determined by experiment or calculated.

For example, one way to calculate Tm of a primer is by reference to the formula for calculating the Tm of primers to a homologous target sequence. This formula is Tm(°C) = 2(A+T) + 4(G+C) - 5. This will provide the Tm under conditions of 3xSSC and 0.1% SDS (where SSC is 0.15M NaCl, 0.015M sodium citrate, pH 7). This formula is generally suitable for primers of up to about 50 nucleotides in length. In the present invention, this formula may be used as an algorithm to calculate a nominal Tm of a primer for a specified sequence derived from a sequence encoding a polypeptide of the invention. The Tm may be compared to a calculated Tm for GPCR sequences of humans and rats, based upon the maximum number of matches to any part of these other sequences.

Suitable conditions for a primer to hybridise to a target sequence may also be measured experimentally. Suitable experimental conditions comprise hybridising a candidate primer to both nucleic acid encoding a polypeptide of the invention and nucleic acid encoding other adenine receptors on a solid support under low stringency hybridising conditions (e.g. 6xSSC at 55°C), washing at reduced SSC and/or higher temperature, for example at 0.2xSSC at 45°C and increasing the hybridisation temperature incrementally to determine hybridisation conditions which allow the primer to hybridise to nucleic acid encoding a polypeptide of the invention but not other purine receptor encoding nucleic acids.

Nucleic acids of the invention, particularly primers, may carry a revealing label. Suitable labels include radioisotopes such as ³²P or ³⁵S, fluorescent labels, enzyme labels, or other protein labels such as biotin. Such labels may be added to polynucleotides or primers of the invention and may be detected using by techniques known per se.

Primers of the present invention may be comprised of synthetic nucleic acids, such as those with modified backbone structures intended to improve stability of the nucleic acid in a cell. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the in vivo activity or lifespan of polynucleotides of the invention.

Also disclosed are antisense sequences based on the nucleic acid sequences described herein, preferably in the form of oligonucleotides, particularly stabilized oligonucleotides, or ribozymes.

Antisense oligonucleotides may be designed to hybridise to the complementary sequence of nucleic acid, pre-mRNA or mature mRNA, interfering with the production of polypeptide encoded by a given target DNA sequence, so that its expression is reduced or prevented altogether. Ribozymes will be designed to cleave mRNA encoded by an adenine binding GPCR encoding nucleic acid sequence of the invention, desirably at a target sequence specific to the adenine binding GPCR, i.e one which is not common to other GPCR sequences. The construction of antisense sequences and their use is described in Peyman and Ulman, Chemical Reviews, 90:543-584, (1990), Crooke, Ann. Rev. Pharmacol. Toxicol., 32:329-376, (1992), and Zamecnik and Stephenson, P.N.A.S, 75:280-284, (1974). The construction of ribozymes and their use is described in for instance Gibson and Shillitoe, Molecular Biotechnology 7(2): 125-137, (1997).

Antisense and ribozyme sequences of the invention may be introduced into mammalian cells lines in culture to study the function of adenine binding GPCR, for example by causing down-regulation of this gene and observing phenotypic effects, or the expression or location of proteins described herein which associate with adenine binding GPCR. In cells where aberrant expression of adenine binding GPCR occurs, such antisense and ribozyme sequences may be used to down-regulate the expression of the gene.

The cDNA sequence of the GPCR of the invention may be cloned using standard PCR (polymerase chain reaction) cloning techniques. This involves making a pair of primers to 5' and 3' ends on opposite strands of SEQ ID NO:1, bringing the primers into contact with mRNA or cDNA obtained from a mammalian cortical cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector. The same applies to SEQ ID NOS:3,5,6,7,8 and 9.

Polynucleotides which are not 100% homologous to the sequence of SEQ ID NO:1 or SEQ ID NO:3 but which encode either SEQ ID NO:2 or SEQ ID NO:4 or other polypeptides of the invention can be obtained in a number of ways.

For example, site directed mutagenesis of the sequence of SEQ ID NO: 1 or SEQ ID NO:3 may be performed. This is useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides. Further changes may be desirable to represent particular coding changes which are required to provide, for example, conservative substitutions.

Nucleic acids of the invention may comprise additional sequences at the 5' or 3' end. For example, synthetic or natural 5' leader sequences may be attached to the nucleic acid encoding polypeptides of the invention. The additional sequences may also include 5' or 3' untranslated regions required for the transcription of nucleic acid of the invention in particular host cells.

In addition, other animal, particularly mammalian (e.g. humans or rabbits), more particularly primate including human, homologues of adenine binding GPCR may be obtained. Such sequences may be obtained by making or obtaining cDNA libraries made from dividing cells or tissues or genomic DNA libraries from other animal species, and probing such libraries with probes comprising all or part of SEQ ID NO:1 or of SEQ ID NO:3 under conditions of medium to high stringency (for example 0.03M sodium chloride and 0.03M sodium citrate at from about 50°C to about 60°C).

The present invention further discloses an isolated DNA sequence comprising sequences encoding a polypeptide of the invention but in which the encoding sequences are divided up into two or more (preferably no more than five, e.g. four or three) exons. Such exon sequences may be natural and obtained from genomic clones, or synthetic. Exon sequences may be used in the construction of mini-gene sequences which comprise nucleic acid encoding polypeptides of the invention which sequences are interrupted by one or more exon sequences.

Mini-genes may also be constructed using heterologous exons, derived from any eukaryotic source.

Nucleic acid according to the present invention, such as a full-length coding sequence or oligonucleotide probe or primer, may be provided as part of a kit, e.g. in a suitable container such as a vial in which the contents are protected from the external environment. The kit may include instructions for use of the nucleic acid, e.g. in PCR and/or a method for determining the presence of nucleic acid of interest in a test sample. A kit wherein the nucleic acid is intended for use in PCR may include one or more other reagents required for the reaction, such as polymerase, nucleosides, buffer solution etc.

The nucleic acid of the invention may be used in nucleic acid-based tests for detecting the adenine binding GPCR encoding sequences in rat, human or other mammalian body or tissues or samples obtained therefrom. In the case of detecting, this may be qualitative and/or quantitative, including such methods as microarray technology on a DNA chip. Detection includes analytical steps such as those which involve sequencing the gene in full or in part.

Such tests for detecting generally comprise bringing a human sample containing DNA or RNA into contact with a probe comprising a nucleic acid of the invention or primer of the invention under hybridizing conditions and detecting any duplex formed between the probe and nucleic acid in the sample. Such detection may be achieved using techniques such as PCR or by immobilizing the probe on a solid support, removing nucleic acid in the sample which is not hybridized to the probe, and then detecting nucleic acid which has hybridized to the probe. Alternatively, the sample nucleic acid may be immobilized on a solid support, and the amount of probe bound to such a support can be detected. Suitable assay methods of this any other formats can be found in for example WO89/03891 and WO90/13667.

A further method of detection according to the invention is in detecting changes to wild-type adenine binding GPCR genes, including single base changes, for example using single stranded conformational polymorphism (SSCP) analysis. Nucleic acid sequence from all or part of an adenine binding GPCR encoding DNA or mRNA in a sample may be hybridized to a reference sequence, and the mobility of the hybrid is observed in a gel under conditions where any non-hybridized regions within the duplex give rise to changes in mobility.

The nucleic acids of the present invention are also useful in tissue distribution studies, to confirm and extend the knowledge of this gene's distribution in the cortex, dorsal root, CNS and PNS and other tissues.

### Polypeptides.

Isolated polypeptides of the invention will be those as defined above in isolated form, free or substantially free of material with which it is naturally associated such as other polypeptides with which it is found in the cell. The polypeptides may of course be formulated with diluents or adjuvants and still for practical purposes be isolated - for example the polypeptides will normally be mixed with gelatin or other carriers if used to coat microtitre plates for use in immunoassays. The polypeptides may be glycosylated, either naturally or by systems of heterologous eukaryotic cells, or they may be (for example if produced by expression in a prokaryotic cell) unglycosylated. Polypeptides may be phosphorylated and/or acetylated.

A polypeptide of the invention may also be in a substantially purified form, in which case it will generally comprise the polypeptide in a preparation in which more than 90%, e.g. 95%, 98% or 99% of the polypeptide in the preparation is a polypeptide of the invention.

Polypeptides of the invention may be modified for example by the addition of histidine residues to assist their purification or by the addition of a signal sequence to promote their secretion from a cell.

Polypeptides having at least 50%, for example 60%, 70%, 80%, 90%, 95% or 98% sequence identity to SEQ ID NO:2 or SEQ ID NO:4 may be polypeptides which are amino acid sequence variants, alleles, derivatives or mutants of SEQ ID NO:2 or SEQ ID NO:4 respectively, and are also disclosed by the present invention. For example such a polypeptide may have an amino acid sequence which differs from that given in SEQ ID NO:2 or SEQ ID NO:4 by one or more of addition, substitution, deletion and insertion of one or more (such as from 1 to 20, for example 2, 3, 4, or 5 to 10) amino acids.

The percentage identity of polypeptide sequences can be calculated using commercially available algorithms which compare a reference sequence (e.g. SEQ ID NO:2 or SEQ ID NO:4 of the present invention) with a query sequence. Further details of assessing identity are described below.

In the present study, the nucleotide sequence SEQ ID NO:2 was analysed by BLAST (Altschul et al., 1997) search to examine whether related GPCRs with known ligands could be found. However the closest homologues of this orphan GPCR that were retrieved, were orphan GPCRs themselves. These were a family of human GPCRs (Derwent sequence database accession numbers Z10067, Z10068, Z10069, Z10070, Z10071 and Z10072) cloned from dorsal root ganglion that have approximately 50% of residues in common with the rat adenine binding GPCR. The present inventors have now identified these six orphan GPCRs as being six human homologues of the rat adenine binding GPCR. The six homologues have been termed human adenine binding GPCR 1, 2, 3, 4, 5 and 6 respectively. The next closest homologue is the mas related gene which shares 33% of residues with adenine binding GPCR.

Where a query sequence is determined to have an identity to that of SEQ ID NO:2 or SEQ ID NO:4 of at least 50% and preferably at least 60%, 70%, 80%, 90%, 95% or 98% said sequence being that of a polypeptide retaining adenine binding receptor activity, such a sequence is also disclosed.

A polypeptide according to the present invention may be isolated and/or purified (e.g. using an antibody) for instance after production by expression from encoding nucleic acid. The isolated and/or purified polypeptide may be used in formulation of a composition, which may include at least one additional component, for example a pharmaceutical composition including a pharmaceutically acceptable excipient, vehicle or carrier.

A polypeptide according to the present invention may be used as an immunogen or otherwise in obtaining specific antibodies. Antibodies are useful in purification and other manipulation of polypeptides, diagnostic screening and therapeutic contexts. This is discussed further below.

A polypeptide according to the present invention may be used in screening for molecules which bind to it or modulate its activity or function. Such molecules may be useful in a therapeutic (possibly including prophylactic) context.

A polypeptide of the invention may be labelled with a revealing label. The revealing label may be any suitable label which allows the polypeptide to be detected. Suitable labels include radioisotopes, e.g. ¹²⁵I, enzymes, antibodies, polynucleotides and linkers such as biotin. Labelled polypeptides of the invention may be used in diagnostic procedures such as immunoassays in order to determine the amount of a polypeptide of the invention in a sample. Polypeptides or labelled polypeptides of the invention may also be used in serological or cell mediated immune assays for the detection of immune reactivity to said polypeptides in animals and humans using standard protocols.

A polypeptide or labelled polypeptide of the invention or fragment thereof may also be fixed to a solid phase, for example the surface of an immunoassay well or dipstick.

Such labelled and/or immobilized polypeptides may be packaged into kits in a suitable container along with suitable reagents, controls, instructions and the like.

Such polypeptides and kits may be used in methods of detection of antibodies to such polypeptides present in a sample or active portions or fragments thereof by immunoassay.

Immunoassay methods are well known in the art and will generally comprise:
(a) providing a polypeptide comprising an epitope bindable by an antibody against said protein;
(b) incubating a biological sample with said polypeptide under conditions which allow for the formation of an antibody-antigen complex; and
(c) determining whether antibody-antigen complex comprising said polypeptide is formed.

### Sequence Identity

The percentage identity of nucleic acid and polypeptide sequences can be calculated using commercially available algorithms which compare a reference sequence with a query sequence. The following programs (provided by the National Center for Biotechnology Information) may be used to determine homologies/identities: BLAST, gapped BLAST, BLASTN and PSI-BLAST, which may be used with default parameters.

The algorithm GAP (Genetics Computer Group, Madison, WI) uses the Needleman and Wunsch algorithm to align two complete sequences that maximizes the number of matches and minimizes the number of gaps. Generally, the default parameters are used, with a gap creation penalty = 12 and gap extension penalty = 4.

Another method for determining the best overall match between a nucleic acid sequence or a portion thereof, and a query sequence is the use of the FASTDB computer program based on the algorithm of Brutlag et al (Comp. App. Biosci., 6; 237-245 (1990)). The program provides a global sequence alignment. The result of said global sequence alignment is in percent identity. Suitable parameters used in a FASTDB search of a DNA sequence to calculate percent identity are: Matrix=Unitary, k-tuple=4, Mismatch penalty=1, Joining Penalty=30, Randomization Group Length=0, Cutoff Score=1, Gap Penalty=5, Gap Size Penalty=0.05, and Window Size=500 or query sequence length in nucleotide bases, whichever is shorter. Suitable parameters to calculate percent identity and similarity of an amino acid alignment are: Matrix=PAM 150, k-tuple=2, Mismatch Penalty=1, Joining Penalty=20, Randomization Group Length=0, Cutoff Score=1 Gap Penalty=5, Gap Size Penalty=0.05, and Window Size=500 or query sequence length in nucleotide bases, whichever is shorter.

### Antibodies

The provision of the polypeptides of the invention enables for the production of antibodies able to bind rat or human adenine binding GPCR in a specific manner. Thus the invention discloses an antibody which is able to bind specifically to a polypeptide of the invention and not to other purinergic GPCRs. Such an antibody will have an affinity for a polypeptide of the invention of at least 100 fold, preferably at least 1000 fold more than to other purinergic GPCRs. Such antibodies may be produced using epitopes of polypeptides of the invention which are not present in other GPCRs. Such epitopes can be determined by seeking differences between polypeptides of the invention and other purinergic GPCR sequences.

Antibodies may be obtained using techniques which are standard in the art. Methods of producing antibodies include immunising a mammal (e.g. mouse, rat, rabbit) with a polypeptide of the invention. Antibodies may be obtained from immunised animals using any of a variety of techniques known in the art, and screened, preferably using binding of antibody to antigen of interest. For instance, Western blotting techniques or immunoprecipitation may be used (Armitage et al, Nature, 357:80-82, 1992).

As an alternative or supplement to immunising a mammal with a peptide, an antibody specific for a protein may be obtained from a recombinantly produced library of expressed immunoglobulin variable domains, e.g. using lambda bacteriophage or filamentous bacteriophage which display functional immunoglobulin binding domains on their surfaces; for instance see WO92/01047.

Antibodies according to the present invention may be modified in a number of ways. Indeed the term "antibody" should be construed as covering any binding substance having a binding domain with the required specificity. Thus the invention covers antibody fragments, derivatives, functional equivalents and homologues of antibodies, including synthetic molecules and molecules whose shape mimics that of an antibody enabling it to bind an antigen or epitope.

Example antibody fragments, capable of binding an antigen or other binding partner are the Fab fragment consisting of the VL, VH, Cl and CH1 domains; the Fd fragment consisting of the VH and CH1 domains; the Fv fragment consisting of the VL and VH domains of a single arm of an antibody; the dAb fragment which consists of a VH domain; isolated CDR regions and F(ab')2 fragments, a bivalent fragment including two Fab fragments linked by a disulphide bridge at the hinge region. Single chain Fv fragments are also disclosed.

The reactivities of antibodies on a sample may be determined by any appropriate means. Tagging with individual reporter molecules is one possibility. The reporter molecules may directly or indirectly generate detectable, and preferably measurable, signals. The linkage of reporter molecules may be direct or indirect, covalent, e.g. via a peptide bond or non-covalent. Linkage via a peptide bond may be as a result of recombinant expression of a gene fusion encoding antibody and reporter molecule.

The mode of determining binding is not a feature of the present invention and those skilled in the art are able to choose a suitable mode according to their preference and general knowledge.

Antibodies according to the present invention may be used in screening for the presence of a polypeptide, for example in a test sample containing cells or cell lysate as discussed, and may be used in purifying and/or isolating a polypeptide according to the present invention, for instance following production of the polypeptide by expression from encoding nucleic acid therefor. Antibodies may modulate the activity of the polypeptide to which they bind and so, if that polypeptide has a deleterious effect in an individual, may be useful in a therapeutic context (which may include prophylaxis).

An antibody may be provided in a kit, which may include instructions for use of the antibody, e.g. in determining the presence of a particular substance in a test sample. One or more other reagents may be included, such as labelling molecules, buffer solutions, elutants and so on. Reagents may be provided within containers which protect them from the external environment, such as a sealed vial.

### Vectors

Nucleic acid sequences of the present invention may be incorporated into vectors, particularly expression vectors. The vector may be used to replicate the nucleic acid in a compatible host cell. Thus in a further embodiment, the invention discloses a method of making polynucleotides of the invention by introducing a polynucleotide of the invention into a replicable vector, introducing the vector into a compatible host cell, and growing the host cell under conditions which bring about replication of the vector. The vector may be recovered from the host cell. Suitable host cells are described below in connection with expression vectors.

Preferably, a polynucleotide of the invention in a vector is operably linked to a control sequence which is capable of providing for the expression of the coding sequence by the host cell, i.e. the vector is an expression vector.

The term "operably linked" refers to a juxtaposition wherein the components described are in a relationship permitting them to function in their intended manner. A control sequence "operably linked" to a coding sequence is ligated in such a way that expression of the coding sequence is achieved under condition compatible with the control sequences.

Suitable vectors can be chosen or constructed, containing appropriate regulatory sequences, including promoter sequences, terminator fragments, polyadenylation sequences, enhancer sequences, marker genes and other sequences as appropriate. Vectors may be plasmids, viral e.g. phage, phagemid or baculoviral, cosmids, YACs, BACs, or PACs as appropriate. Vectors include gene therapy vectors, for example vectors based on adenovirus, adeno-associated virus, retrovirus (such as HIV or MLV) or alpha virus vectors.

The vectors may be provided with an origin of replication, optionally a promoter for the expression of the said polynucleotide and optionally a regulator of the promoter. The vectors may contain one or more selectable marker genes, for example an ampicillin resistance gene in the case of a bacterial plasmid or a neomycin resistance gene for a mammalian vector. Vectors may be used in vitro, for example for the production of RNA or used to transfect or transform a host cell. The vector may also be adapted to be used in vivo, for example in methods of gene therapy. Systems for cloning and expression of a polypeptide in a variety of different host cells are well known. Suitable host cells include bacteria, eukaryotic cells such as mammalian and yeast, and baculovirus systems. Mammalian cell lines available in the art for expression of a heterologous polypeptide include Chinese hamster ovary cells, HeLa cells, baby hamster kidney cells, COS cells and many others.

Promoters and other expression regulation signals may be selected to be compatible with the host cell for which the expression vector is designed. For example, yeast promoters include *S*. *cerevisiae* GAL4 and ADH promoters, *S*. *pombe* nmt1 and adh promoter. Mammalian promoters include the metallothionein promoter which can be induced in response to heavy metals such as cadmium. Viral promoters such as the SV40 large T antigen promoter or adenovirus promoters may also be used. All these promoters are readily available in the art.

The vectors may include other sequences such as promoters or enhancers to drive the expression of the inserted nucleic acid, nucleic acid sequences so that the polypeptide is produced as a fusion and/or nucleic acid encoding secretion signals so that the polypeptide produced in the host cell is secreted from the cell.

Vectors for production of polypeptides of the invention of for use in gene therapy include vectors which carry a mini-gene sequence of the invention.

For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1992.

Vectors may be transformed into a suitable host cell as described above to provide for expression of a polypeptide of the invention. Thus, in a further aspect the invention discloses a process for preparing polypeptides according to the invention which comprises cultivating a host cell transformed or transfected with an expression vector as described above under conditions to provide for expression by the vector of a coding sequence encoding the polypeptides, and recovering the expressed polypeptides. Polypeptides may also be expressed in vitro systems, such as reticulocyte lysate.

A further embodiment of the invention discloses host cells transformed or transfected with the vectors for the replication and expression of polynucleotides of the invention. The cells will be chosen to be compatible with the said vector and may for example be bacterial, yeast, insect or mammalian. The host cells may be cultured under conditions for expression of the gene, so that the encoded polypeptide is produced. If the polypeptide is expressed coupled to an appropriate signal leader peptide it may be secreted from the cell into the culture medium. Following production by expression, a polypeptide may be isolated and/or purified from the host cell and/or culture medium, as the case may be, and subsequently used as desired, e.g. in the formulation of a composition which may include one or more additional components, such as a pharmaceutical composition which includes one or more pharmaceutically acceptable excipients, vehicles or carriers

Polynucleotides according to the invention may also be inserted into the vectors described above in an antisense orientation in order to provide for the production of antisense RNA or ribozymes.

### Assays

The present invention also discloses an assay for a compound capable of interacting with adenine binding GPCR polypeptides of the present invention, which assay comprises: providing a G protein coupled receptor comprising at least one polypeptide according to the invention, contacting the receptor with a putative binding compound; and determining whether said compound is able to interact with said receptor.

In one embodiment of the assay, the receptor or subunits of the receptor may be employed in a binding assay. Binding assays may be competitive or non-competitive. Such an assay can accommodate the rapid screening of a large number of compounds to determine which compounds, if any, are capable of binding to the polypeptides. Subsequently, more detailed assays can be carried out with those compounds found to bind, to further determine whether such compounds act as agonists or antagonists of the polypeptides of the invention.

The present invention still further discloses a bioassay for identifying compounds which modulate the activity of receptors comprising polypeptides of the invention. In one embodiment, the bioassay is conducted using Saccharomyces cerevisiae cells, engineered with GPCRs of the invention and rat or human G proteins to replace the endogenous GPCRs and G proteins, which normally function as the pheromone pathway required for conjugation and mating of the yeast cells. The normal yeast response to activating ligand (growth arrest) is also engineered to be converted to positive growth. With such engineering, the yeast cells can be used in functional assays to identify agonists and antagonists to the GPCRs of the invention, with growth indicating activation of the GPCR. For example, the bioassay may be conducted by contacting such yeast cells expressing the GPCR comprising a polypeptide of the invention with at least one potential agonist and thereafter monitoring the cells for changes in growth activity. In yet another embodiment, the bioassay is conducted by contacting such yeast cells expressing receptor comprising a polypeptide of the invention with a constant amount of known agonist e.g. adenine and increasing amounts of at least one potential antagonist and thereafter monitoring the cells for changes in growth.

The present invention also discloses a bioassay for identifying compounds which modulate the regulatory regions of the adenine binding GPCR gene. Such an assay is conducted utilising rat or human cells capable of expressing a polypeptide of the invention (preferably of SEQ ID NO:2 or SEQ ID NO:4). The cells are contacted with at least one compound wherein the ability of said compound to modulate the regulatory region is known. Thereafter, the cells are monitored for expression of the nucleic acid of the invention. Alternatively, the promoter may be linked to a reporter gene. Suitable reporter genes that may be employed include, for example, the chloramphenicol acetyltransferase gene, the luciferase gene, and the like.

A compound or a signal that "modulates the activity" of a polypeptide of the invention refers to a compound or a signal that alters the activity of the polypeptide so that it behaves differently in the presence of the compound or signal than in the absence of the compound or signal. Compounds affecting modulation include agonists and antagonists. An agonist encompasses a compound such as adenine which activates adenine binding GPCR function. Alternatively, an antagonist includes a compound that interferes with adenine binding GPCR function. Typically, the effect of an antagonist is observed as a blocking of agonist-induced receptor activation. Antagonists include competitive as well as non-competitive antagonists. A competitive antagonist (or competitive blocker) interacts with or near the site specific for agonist binding. A non-competitive antagonist or blocker inactivates the function of the receptor by interacting with a site other than the agonist interaction site. As described below, guanine has a weak antagonistic effect at these receptors.

As understood by these of skill in the art, bioassay methods for identifying compounds that modulate the activity of receptors such as polypeptides of the invention generally require comparison to a control. One type of "control" is a cell or culture that is treated substantially the same as the test cell or test culture exposed to the compound, with the distinction that the "control" cell or culture is not exposed to the compound. Another type of "control" cell or culture that can be employed is a cell or culture that is identical to transfected cells, the exception that the "control" cell or culture does not express functional adenine binding GPCR. Accordingly, the response of the transfected cell can be compared with that of the "control" cell or culture to the same compound under the same reaction conditions.

Particularly preferred types of assays include binding assays and functional assays which may be performed as follows:

### Binding assays

Over-expression of nucleic acid encoding polypeptides of the invention in cell lines (including mammalian HEK 293 cells, CHO cells and Sf9 insect cells) may be used to produce membrane preparations bearing said polypeptides (referred to in this section as adenine binding GPCR for convenience) for ligand binding studies. These membrane preparations can be used in conventional filter-binding assays (eg. Using Brandel filter assay equipment) or in high throughput Scintillation Proximity type binding assays (SPA and Cytostar-T flashplate technology; Amersham Pharmacia Biotech) to detect binding of radio-labelled purinergic ligands (including [2-³H Adenine (Amersham, TRK311 and 8-³H Adenine (Amersham, TRK343] and displacement of such radio-ligands by competitors for the binding site. Radioactivity can be measured with Packard Topcount, or similar instrumentation, capable of making rapid measurements from 96-, 384-, 1536- microtitre well formats. SPA/Cytostar-T technology is particularly amenable to high throughput screening and therefore this technology is suitable to use as a screen for compounds able to displace standard ligands.

Another approach to study binding of ligands to adenine binding GPCR protein in an environment approximating the native situation makes use of a surface plasmon resonance effect exploited by the Biacore instrument (Biacore). Adenine binding GPCR in membrane preparations or whole cells could be attached to the biosensor chip of a Biacore and binding of ligands examined in the presence and absence of compounds to identify competitors of the binding site.

### Functional assays

Since adenine binding GPCR acts via Gᵢ or Go(inhibitory G protein), which usually interacts with GIRK (inward rectifying potassium channels), potassium ion flux should result on activation of these receptors. This flux of ions may be measured in real time using a variety of techniques to determine the agonistic or antagonistic effects of particular compounds. Therefore, recombinant adenine binding GPCR receptors expressed in cell lines or, for example, *Xenopus* oocytes can be characterised using whole cell and single channel electrophysiology to determine the mechanism of action of compounds of interest. Electrophysiological screening, for compounds active at adenine binding GPCR, may be performed using conventional electrophysiological techniques and when they become available, novel high throughput methods currently under development.

Fluorescence - Calcium and sodium fluxes are measurable using several ion-sensitive fluorescent dyes, including fluo-3, fluo-4, fluo-5N, fura red, Sodium Green, SBFI and other similar probes from suppliers including Molecular Probes. Calcium and sodium influx as a result of adenine binding GPCR can thus be characterised in real time, using fluorometric and fluorescence imaging techniques, including fluorescence microscopy with or without laser confocal methods combined with image analysis algorithms.

Another approach is a high throughput screening assay for compounds active as either agonists or modulators which affect calcium transients. This assay is based around an instrument called a **FL**uorescence **I**maging **P**late **R**eader ((FLIPR®), Molecular Devices Corporation). In its most common configuration, it excites and measures fluorescence emitted by fluorescein-based dyes. It uses an argon-ion laser to produce high power excitation at 488 nm of a fluorophore, a system of optics to rapidly scan the over the bottom of a 96-/384-well plate and a sensitive, cooled CCD camera to capture the emitted fluorescence. It also contains a 96-/384-well pipetting head allowing the instrument to deliver solutions of test agents into the wells of a 96-/384-well plate. The FLIPR assay is designed to measure fluorescence signals from populations of cells before, during and after addition of compounds, in real time, from all 96-/384-wells simultaneously. The FLIPR assay may be used to screen for and characterise compounds functionally active at recombinant adenine binding GPCR, eg rat or human adenine binding GPCR, expressed in cell lines. As described below, calcium transients in cells transfected with adenine binding GPCR were measured using the FLIPR assay in order to measure activation of the receptors by various substrates in order to determine the natural ligand of the receptor.

Compounds found to modulate the activity of the polypeptides of the present invention have a number of therapeutic uses. As is obvious from the BLAST searches adenine binding GPCR does not belong to any of the known subfamilies of GPCRs that are activated structures similar to adenine. This suggests that adenine binding GPCR is a member of a new subgroup of purinergic GPCRs where it will be possible to develop a novel pharmacology for the different therapeutic areas in which purinergics have been applied up to now. In specific, this includes diseases related to apoptosis (Chow et al., 1997), pathological tremor disorders (Mally and Stone, 1996), nociception (Burnstock, 1997). Purinergic compounds have also been suggested for use as anticonvulsives, sedatives, hypnotics, ataxic (CNS), hypotensives, antiarrhythmics, negative chronotropics, dromotropics and inotropics, inhibitors of platelet aggregation, stimulators of NO production by vascular endothelial cells in the cardiovascular system and as inhibitors of gastric secretion (Gil-Rodrigo et al, Pharmacol. Res. 22(2):103-13, 1990). Furthermore studies based on transgenic animals suggest that receptors from this family play a role in bronchoconstriction, vasodilation, inflammation and facilitation of neurotransmission (Nyce, 1999). In summary adenine binding GPCR and related receptors can be used as a valuable tool for drug development in a variety of therapeutic areas.

Furthermore the demonstration that adenine itself acts as the natural ligand to this receptor opens up the possibility of the use of adenine and compounds mimicking or antagonising adenine in the development of treatments for the diseases mentioned above.

### Tissue distribution studies

Adenine binding GPCR DNA sequences are useful for confirming and extending knowledge of this GPCR's distribution by hybridisation/PCR studies in human health and disease. Recombinant adenine binding GPCR in cell lines are useful for characterising adenine binding GPCR function and a variety of biochemical and biophysical methods are available for assaying this receptor. Assays of the invention described herein are also useful for drug discovery screening purposes.

### Binding agents

Thus the invention further discloses novel binding agents, including modulatory agents obtained by an assay according to the present invention, and compositions comprising such agents. Agents which bind to the receptor and which may have agonist or antagonist activity may be used in methods of treating diseases whose pathology is characterised by action via the adenine binding GPCR receptor, and such use forms a further aspect of the invention. Such diseases may include diseases related to apoptosis, pathological tremor disorders and nociception bronchoconstriction, vasodilation, inflammation and facilitation of neurotransmission. Furthermore, the agents may be used in treatments where anticonvulsives, sedatives, hypnotics, ataxic (CNS), hypotensives, antiarrhytmics, negative chronotropics, dromotropics and inotropics, inhibitors of platelet aggregation, stimulators of NO production by vascular endothelial cells in the cardiovascular system or as inhibitors of gastric secretion may be useful. The agents may be administered an effective amount of an agent of the invention. Since many of the above-mentioned conditions are chronic and often incurable, it will be understood that "treatment" is intended to include achieving a reduction in the symptoms for a period of time such as a few hours, days or weeks, and to include slowing the progression of the course of the disease.

Such agents may be formulated into compositions comprising an agent together with a pharmaceutically acceptable carrier or diluent. The agent may in the form of a physiologically functional derivative, such as an ester or a salt, such as an acid addition salt or basic metal salt, or an N or S oxide. Compositions may be formulated for any suitable route and means of administration. Pharmaceutically acceptable carriers or diluents include those used in formulations suitable for oral, rectal, nasal, inhalable, topical (including buccal and sublingual), vaginal or parenteral (including subcutaneous, intramuscular, intravenous, intradermal, intrathecal and epidural) administration. The choice of carrier or diluent will of course depend on the proposed route of administration, which, may depend on the agent and its therapeutic purpose. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

For solid compositions, conventional non-toxic solid carriers include, for example, pharmaceutical grades of mannitol, lactose, cellulose, cellulose derivatives, starch, magnesium stearate, sodium saccharin, talcum, glucose, sucrose, magnesium carbonate, and the like may be used. The active compound as defined above may be formulated as suppositories using, for example, polyalkylene glycols, acetylated triglycerides and the like, as the carrier. Liquid pharmaceutically administrable compositions can, for example, be prepared by dissolving, dispersing, etc, an active compound as defined above and optional pharmaceutical adjuvants in a carrier, such as, for example, water, saline aqueous dextrose, glycerol, ethanol, and the like, to thereby form a solution or suspension. If desired, the pharmaceutical composition to be administered may also contain minor amounts of non-toxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see Remington's Pharmaceutical Sciences, Mack Publishing Company, Easton, Pennsylvania, 15th Edition, 1975.

The composition or formulation to be administered will, in any event, contain a quantity of the active compound(s) in an amount effective to alleviate the symptoms of the subject being treated.

Dosage forms or compositions containing active ingredient in the range of 0.25 to 95% with the balance made up from non-toxic carrier may be prepared.

For oral administration, a pharmaceutically acceptable non-toxic composition is formed by the incorporation of any of the normally employed excipients, such as, for example, pharmaceutical grades of mannitol, lactose, cellulose, cellulose derivatives, sodium crosscarmellose, starch, magnesium stearate, sodium saccharin, talcum, glucose, sucrose, magnesium, carbonate, and the like. Such compositions take the form of solutions, suspensions, tablets, pills, capsules, powders, sustained release formulations and the like. Such compositions may contain 1%-95% active ingredient, more preferably 2-50%, most preferably 5-8%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

The percentage of active compound contained in such parental compositions is highly dependent on the specific nature thereof, as well as the activity of the compound and the needs of the subject. However, percentages of active ingredient of 0.1% to 10% in solution are employable, and will be higher if the composition is a solid which will be subsequently diluted to the above percentages. Preferably, the composition will comprise 0.2-2% of the active agent in solution.

### EXAMPLES

The following examples illustrate the invention. Other embodiments will occur to the person skilled in the art in light of these examples.

### Example 1 Analysis of Novel GPCR

The sequence described in SEQ ID NO:1 was analysed by BLAST (Altschul et al., 1997) search to examine whether related GPCRs with known ligands could be found. However the closest homologues of this orphan GPCR that were retrieved, were orphan GPCRs themselves. These were a family of human GPCRs (Derwent sequence database accession numbers Z10067, Z10068, Z10069, Z10070 Z10071 and Z10072) cloned from dorsal root ganglion that have approximately 50% of residues in common with the novel GPCR (rat adenine binding GPCR). The next closest homologue is the mas related gene which shares 33% of residues with adenine binding GPCR.

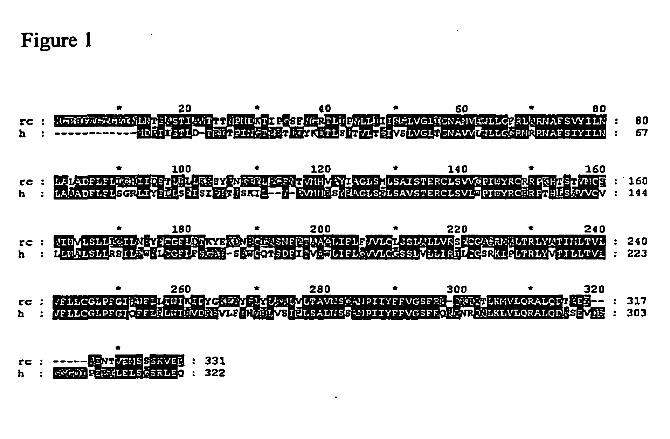

Using the nucleotide sequence of SEQ ID NO:1, the present inventors have identified the human GPCRs above as being six human homologues of the rat novel GPCR. These have been termed human adenine binding GPCR1 (SEQ ID NO:3), human adenine binding GPCR2 (SEQ ID NO:5), human adenine binding GPCR3 (SEQ ID NO:6), human adenine binding GPCR4 (SEQ ID NO:7), human adenine binding GPCR5 (SEQ ID NO:8) and human adenine binding GPCR6 (SEQ ID NO:9). The nucleotide and amino acid sequences for human adenine binding GPCR1 are shown in SEQ ID NO:3 and SEQ ID NO:4 with the amino acid sequences of rat adenine binding GPCR (SEQ ID NO:2) and human adenine binding GPCR.1 (SEQ ID NO:4) aligned in Figure 1. The nucleic acid sequence for human adenine binding GPCR2, human adenine binding GPCR3, human adenine binding GPCR4, human adenine binding GPCR5 and human adenine binding GPCR6 shown in SEQ ID NOs: 5-9 respectively. "N" in SEQ ID NO: 5 is A or T or C or G.

### Example 2 Identification of the natural ligand of the novel GPCR

As no related GPCRs with known ligands could be found in the prior art, the inventors set about trying to identify which natural ligand would bind to the receptor. In order to identify the natural ligand of the sequenced GPCR, 'reverse pharmacology' was employed. Essentially an extract from the tissue where this GPCR is expressed (brain cortex) was prepared and the purification of the natural ligand was followed by a cellular assay based on the activation of the orphan GPCR.

### Example 3 Transient expression in mammalian cells and FLIPR assay

The full length reading frame was inserted into the mammalian expression vector pcDNA3 (Invitrogen, Carlsbad, CA, U.S.A.) and the resulting expression construct was transiently transfected with the FuGENE 6 reagent (Roche Molecular Biochemicals, Mannheim, Germany) into HEK293 cells stably transfected (E. Bender, unpublished) with pLEC/Gqi or pLEC/Gqo (Molecular Devices, Sunnyvale, CA, U.S.A.). The cells were loaded with Fluo-4 (Molecular Probes, Eugene, OR, U.S.A.) according to the recommendations of the supplier.

### Example 4 Preparation of tissue extracts and extract fractionation

10 kg porcine cortex were homogenized and extracted in methanol/water/acetic acid, 90/9/1, v/v/v. After centrifugation, the supernatant was delipidated by n-hexane extraction and the aqueous layer was fractionated by a Megabondelute fractionation. The material eluting at 50% acetonitrile/H₂O was further fractionated on a HPLC C18 column. The fractions derived thereof were tested for activation of the novel GPCR in the FLIPR assay. Subsequent purification steps on an Hypersil C18, an analytical C8 column, a Biosep column and finally an HPLC C18 column were also followed by the FLIPR based activity assay for fractions activating the novel GPCR transfected cells. The fraction that contained the activity after the final purification step was analysed by mass spectrometry (Q-TOF, MicroMass, Manchester, U.K.).

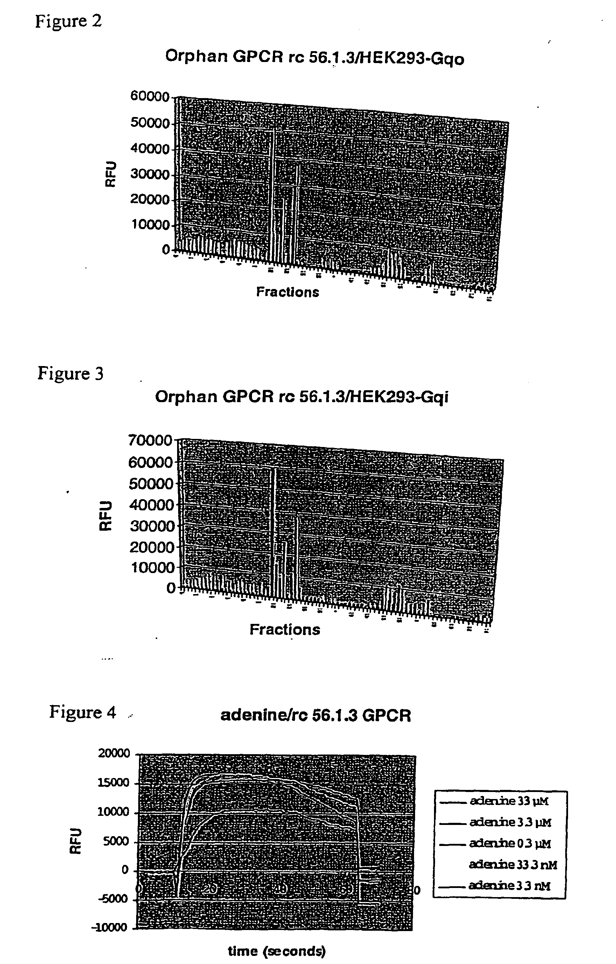

The screening of the porcine cortex extract after the first fractionation on a C18 column delivered a rather broad range of fractions that resulted in transient intracellular Ca²⁺ release for cells transiently transfected with the novel GPCR expression construct. This was the case for HEK293 cells expressing either the Gqo or the Gqi chimera (Figure 2 and Figure 3), but not for wild-type HEK293 cells (data not shown). One of these fractions, number 19 that yielded for both G protein chimeras a strong stimulation was chosen for further subfractionation and processed as described above. The purification was guided by the FLIPR based activity assay and the fraction that showed activity from the last column run was subjected to mass spectrometry to determine purity as well as the structure of the compounds that it contained.

### Example 5 Identification of activating substance by mass spectrometry

Mass analysis of the active purified fraction from the Symmetry C18 (4.6 x 250 mm; 5µm, Waters) reverse phase column gave one major ion at m/z 136. The mass was locked on the (NaI+Na)⁺ ion (172.8840 Da) giving a more accurate mass for the ion of interest (136.0623 Da). This mass was used in the elemental composition analysis. The mass accuracy tolerance was set at 20 ppm. The best match was given by C₅H₆N₅ (136.0623 Da), corresponding to Adenine + H⁺. The CID spectrum of the native molecule was compared to that of synthetic adenine.

Fragmentation of both parent molecules resulted in the same fragment ions at m/z 119.0, 109.0, 94.0, 92.0, 82.0, 77.0, 67.0 and 65.0. The conclusion from the results obtained by tandem mass spectrometry on a Q-TOF was that the activating substance was adenine. The novel GPCR was therefore named adenine binding GPCR.

### Example 6 Testing of novel GPCR with selected pure compounds

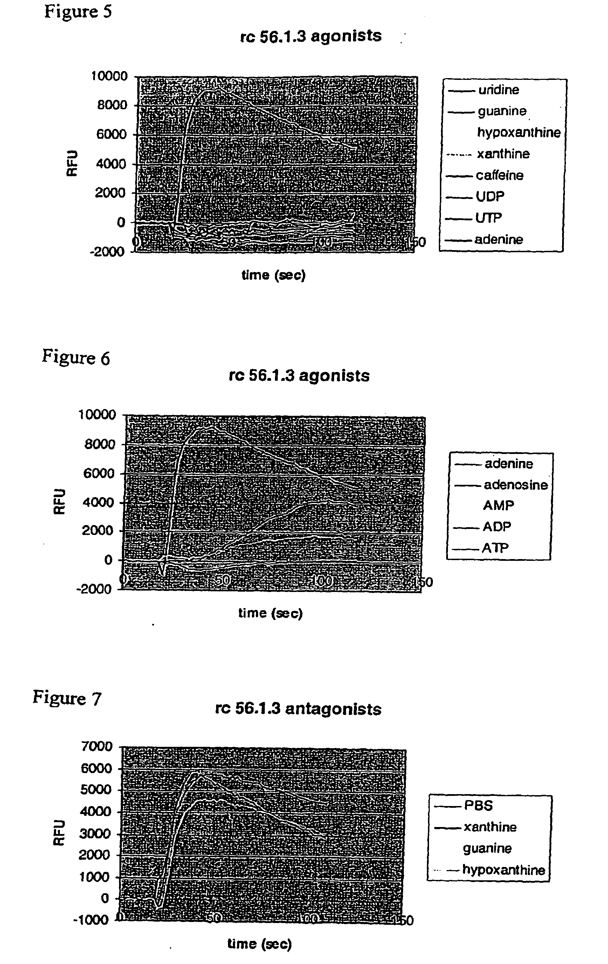

To confirm the result obtained from mass spectrometry pure adenine was dissolved in PBS to give final concentrations 3.3nM - 33µM and added to HEK293/Gaqo cells transiently transfected with the rat novel GPCR(adenine binding GPCR)/pcDNA3 expression construct and to those transfected with empty expression vector. Using the FLIPR assay as above, calcium transients were measured in both sets of cells with the transients attributable to the novel GPCR measured as the difference between the two transients. Results are illustrated in Figure 4. Adenine provoked a dose responsive calcium transient in the cells, indicating that the novel GPCR (adenine binding GPCR) has a high affinity for this compound. In contrast, other purines and pyrimidines did not demonstrate agonistic activity. Figure 5 illustrates that calcium transients could not be elicited in the HEK293/Gaqo cells by the addition of 3.3µM uridine, guanine, hypoxanthine, xanthine, caffeine, UDP or UDP, confirming the specificity of the adenine binding GPCR receptor for adenine. To further test the specificity of the receptor for adenine, a number of nucleoside and nucleotide derivatives were tested using the same assay. The results are shown in Figure 6. For the nucleoside and nucleotide derivatives of adenine only adenosine and AMP show some weak partial agonism at high concentrations (> 3.3 • M).

In order to examine whether among the same compounds, molecules with antagonistic properties could be found, cells transfected with adenine binding GPCR were exposed to various purines (guanine, hypoxanthine, xanthine, caffeine, theophylline), pyrimidines (uridine, UDP, UTP), known antagonists at other nucleotide receptors (suramin, reactive blue) and nucleoside or nucleotide derivatives of adenine (adenosine, AMP, ADP, ATP) at concentrations ranging from 30 µM to 30 pM while measuring Ca2+ transients in the FLIPR. No further agonistic effects besides those mentioned above were discovered in this experiment. After a 15 minutes incubation at room temperature, the same cells were challenged with 30 nM adenine, to detect antagonistic properties of molecules added in the first round of superfusion. Only guanine showed a weak antagonistic effect at 30 and 3 µM (Figure 7). All other compounds did not elicit antagonistic effects on adenine binding GPCR at the tested concentrations.

### Examples 7 to 12 Characterization of the rat Adenine receptor on transiently and permanently transfected CHO cells

To further characterise the novel rat Adenine receptor, CHO cells were transiently and permanently transfected using the lipofectamine plus method. The pharmacological properties of a variety of adenine-derivatives were evaluated using radioligand binding experiments, stimulation of [³⁵S]GTPγS binding and cAMP measurements. All these experiments were run in parallel with wild type CHO cells as a control.

Example 7 describes radioligand binding experiments using [³H] adenine in transiently and permanently transfected CHO cells. Measurement of binding of [³⁵S] GTPγS in response to adenine is described for transiently and permanently transfected cells in Examples 8 and 10 respectively. Measurement of cAMP in response to adenine is described for transiently and permanently transfected cells in examples 9 and 11 respectively. In example 12, the effect of adenine on intracellular Ca²⁺ is described.

### Cell Culture and Transfection Methods

CHO cells were maintained in Dulbecco's modified Eagle's medium (DMEM)/nutrient mixture Ham's F12 (Life Technologies, Belgium) in a ratio of 1:1 supplemented with 10% heat inactivated fetal calf serum, and an antibiotic solution containing 100 IU/ml penicillin G, 100 µg/ml streptomycin sulfate, 110 µg/µl pyruvic acid and 300 µg/ml L-glutamine. The cells were grown in 175 cm² culture flasks at 37ºC in a 5% CO₂ environment. For the permanently transfected cells, once every two weeks, the transfected cells were cultured in a selection medium (Dulbecco's modified Eagle's medium (DMEM) nutrient mixture Ham's F12 (ratio 1:1), Life Technologies, Belgium) containing 800 µg/ml geneticine (G418).

For transient transfections of CHO cells the lipofectamine PLUS (Life Technologies) method was used. One day before the transfection, the CHO cells were seeded out in 50 mm petri dishes at a density of 20,000 cells/cm² in DMEM nutrient mixture Ham's F12 (ratio 1:1) (Life Technologies). For the transfection of one petri dish, 6.5 µg DNA was added to 17.5 µl PLUS reagent (Life Technologies), mixed and incubated for 15 minutes at room temperature (solution A). 15 µl of lipofectamine PLUS reagent was added to 735 µl serum free medium (solution B). Solution A and B were combined, mixed gently and incubated for 15 minutes at room temperature to form a complex. After 15 minutes, 3.5 ml serum-free medium was added to the mix. The cells were washed once with serum-free medium and the DNA-lipofectamine PLUS complex (in a final volume of 5 ml) was added onto the cells and incubated for 3 hours at 37°C in a 5% CO₂ incubator. After the incubation, 5 ml of DMEM/Ham's F12 (ratio 1:1), supplemented with 20% heat inactivated fetal calf serum, was added onto the cells and left overnight in the incubator. The next day, the medium was removed and replaced with the same growth medium as above.

The lipofectamine PLUS method was also used for permanent transfection of CHO cells with slight modifications. One day after the transfection, the medium was replaced with selection medium which was replaced every 2 to 3 days. The cells were grown till colonies appeared (between day 8 and 10) and split to a 175 cm² culture flask for further culture and experiments. Monoclonal cell lines were prepared by limited dilution seeding in 96 well plates.

### Membrane preparation

The membranes were prepared as total particulate fractions. The cell lines were cultured to 90% confluency on 145 mm petri dishes and treated with 5 mM sodium butyrate, 24 hours before collection. The culturing medium was removed and the cells were washed twice with ice cold phosphate buffered saline (PBS without Ca²⁺ and Mg²⁺), scraped from the plates in 50 mM Tris-HCl buffer, pH 7.4, and collected by centrifugation (10 minutes at 16,000 RPM at 4ºC). The cell pellet was re-suspended in hypotonic 5 mM Tris-HCl buffer, pH 7.4, and homogenized with an Ultra Turrax homogenizer (Janker Kunkel, Germany). The homogenate was centrifuged at 18,000 RPM for 20 minutes at 4ºC. The final pellet was re-suspended in 50 mM Tris-HCl buffer, pH 7.4 and stored in aliquots at - 70ºC. A protein determination was performed using the Bradford protein assay (Biorad, Germany) using bovine serum albumin (BSA) as standard.

### Example 7 [³ H]Adenine binding

A) Methods - [³H] Adenine binding experiments were performed to characterize wild type cell lines before transfection as well as transiently and permanently transfected cell lines. Membranes were thawed on ice and diluted in 50 mM HEPES buffer, pH 7.4 supplemented with 10 mM MgCl₂ and 1 mM EGTA. Non-specific binding was defined in the presence of 1 µM adenine and a 1 hour incubation at 25°C with [³H]adenine (specific activity of 30.4 Ci/mmol), was found to be optimal for saturation and competition binding assays. Both assays were carried out in a final volume of 500 µl, using 18 µg of membrane protein for the transfected CHO cells and 30 µg of membrane protein for the wild type CHO cells for the saturation binding. For competition binding experiments, 15 and 50 µg of membrane protein was used for the transfected and wild type cells, respectively. The reaction was terminated by rapid filtration through Whatman GF/B filters using a Brandel multi-channel cell harvester (96 wells). The filters were washed three times with 3 ml ice cold 50 mM HEPES buffer, pH 7.4, transferred to liquid scintillation vials and 3 ml of scintillation fluid (Ultima Gold MV, Packard, Netherlands) was added. Samples were counted in a β-scintillation counter after at least 6 hours to permit the glass fiber filters to become uniformly translucent.
Saturation analysis of [³H]adenine binding was carried out using 18 concentrations of the radioligand ranging from 0.2 to 90 nM.
Competitive inhibition of [³H]adenine by the compounds, adenine, 1-methyladenine and other analogues was performed with 15 nM of [³H]adenine and various concentrations of the unlabeled compounds, spanning 5 orders of a magnitude.
The maximum number of binding sites (Bₘₐₓ), the apparent equilibrium dissociation constant (K_{D}) and the pIC₅₀ values (as IC₅₀ value represents the concentration that inhibits 50% of specific radioligand binding) were derived from non-linear curve fitting.
b) Results: Preliminary radioligand binding experiments with [³H]adenine were conducted on different wild-type cells. The assay conditions were identical to those for the transfected cells. Binding results obtained with the different cell lines are shown below:

| | |
|---|---|
| C6 glioma: | 839 fmoles/mg protein |
| Cos7: | 226 fmoles/mg protein |
| HEK293: | 168 fmoles/mg protein |
| NIH3T3: | 642 fmoles/mg protein |
| CHO: | 303 fmoles/mg protein (pool 1) |
| | 583 fmoles/mg protein (pool 2) |

The CHO cells were chosen for transfection with the rat adenine receptor and further experiments.

In independent experiments 48 hours post transfection (efficiency was 50-60 % as evaluated by β-gal staining using conventional techniques known to the skilled person) CHO cells were evaluated for [³H]adenine binding. In preliminary binding experiments performed using 4 nM [³H]adenine, the following binding levels were observed:

| | |
|---|---|
| pool 1 | 3554 fmoles/mg protein |
| pool 2 | 3444 fmoles/mg protein |
| pool 3 | 2436 fmoles/mg protein |
| pool 4 | 1717 fmoles/mg protein |

Saturation binding analysis of [³H]adenine, was carried out with and without addition of 100 •M guanylylimidodiphosphate (Gpp-NHp) on the transfected as well as on the wild type CHO cells, using an average of 16 radioligand concentrations ranging from 0.1 to 80 nM (table 1). Addition of Gpp-NHp blocks the high-affinity or agonist binding sites of GPCRs, so that only low-affinity sites are available.

In transfected cells average saturation binding levels were around 19 pmol/mg and the K_{D}-value for [³H]adenine was 24 nM. Specific binding with similar K_{D} was also observed in wild type CHO cells, but with a much lower Bmax (- 1.1 pmol/mg). Furhermore, the K_{D} of transfected cells was sensitive to Gpp-NHp addition, whereas the K_{D} in wild type cells was not.

Competitive inhibition of [³H]adenine binding by a variety of adenine derivatives was performed using 15 nM of [³H]adenine on cells which had been transiently transfected with the receptor and on the wild type CHO cells (table 2).

High affinity competition binding could only be obtained with adenine itself, with a Ki-value of 18 nM, which is very close to the directly measured K_{D}-value.

### Example 8 [³⁵S]GTP•S binding in transiently infected cells

a) Methods - Determination of [³⁵S]GTP•S binding to the G-proteins was carried out according to a modified procedure of Lazareno (Methods Mol. Biol., 83:107-116, 1997). These experiments were performed for a first functional evaluation of the receptor on the level of a membrane preparation.
   In preliminary [³¹S]GTPγS binding experiments, assay conditions were optimized, which resulted in the choice of the following incubation buffer: 20 mM Hepes, pH 7.4 containing 100 mM NaCl, 10 •M GDP, 10 mM MgCl₂ and 10 µg/ml saponine. The assay mixture contained 10 and 35 µg of membrane protein for the transfected and wild type CHO cells, respectively.
   For testing agonistic activity, 175 µl of diluted membranes was pre-incubated in the buffer described above together with 25 µl of buffer and 25 µl of varying concentrations of the compound in a total volume of 225 µl. After a 20 minute pre-incubation period at 30ºC, 25 µl of [³⁵S]GTPγS was added to a final concentration of 0.25 nM and the assay mixtures were further incubated for 20 minutes at 30°C. Bound and free [³⁵S]GTPγS were separated by rapid filtration over an UniFilter^{™}-96, GF/B^{™} under reduced pressure using Filtermate 196 filtration (Packard Company (Netherlands). The amount of bound radioactivity on the filter unit was determined after drying of the filters and scintillant addition (Microscint-O; Packard Company) by liquid scintillation counting.
   Basal [³⁵S]GTPγS binding was measured in absence of compounds. Stimulation by agonist was calculated as the percentage increase above basal levels. The sigmoid agonist concentration-response curves were analyzed by non-linear regression using the GraphPad Prism program. The different concentration points were run in duplicates and the reported data were retrieved from independent experiments.
b) Results - [³⁵S]GTPγS binding on membranes from transiently transfected CHO cells had a maximum stimulation by adenine of 150% above basal binding levels. The mean EC₅₀ for adenine was 112 nM. 1-methyladenine and AMP stimulated the receptor partially with a much lower potency, in correspondence with their lower affinity in competition binding assays.

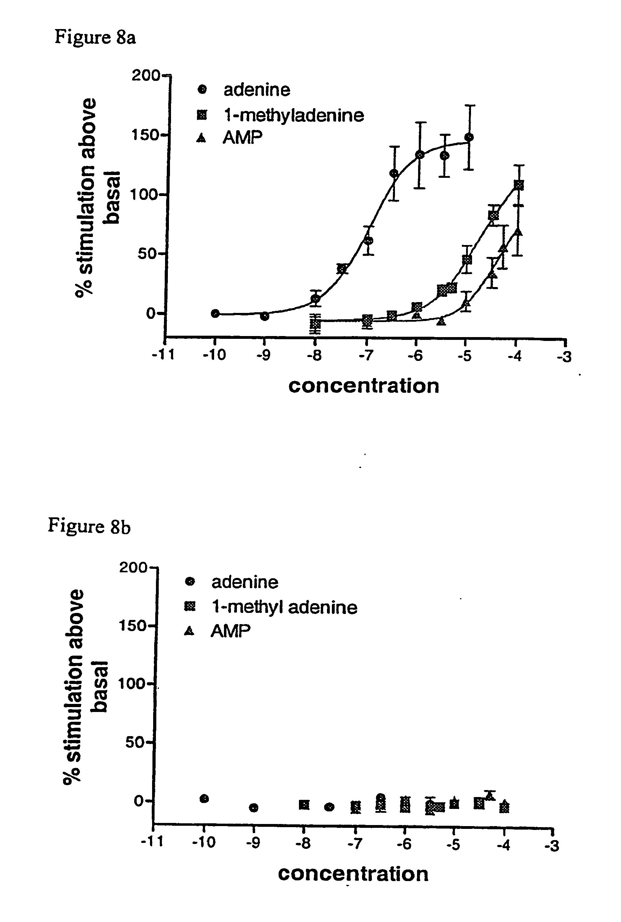

No stimulation of GTPγS binding was measured in wild type CHO cells (see table 3 and Figure 8a (transiently transfected CHO) and Figure 8b(wild type CHO)). The values in percentages are normalized to maximum adenine response, i.e. the adenine stimulation over basal levels is taken as 100%.

### Example 9 cAMP measurements in transiently transfected CHO cells

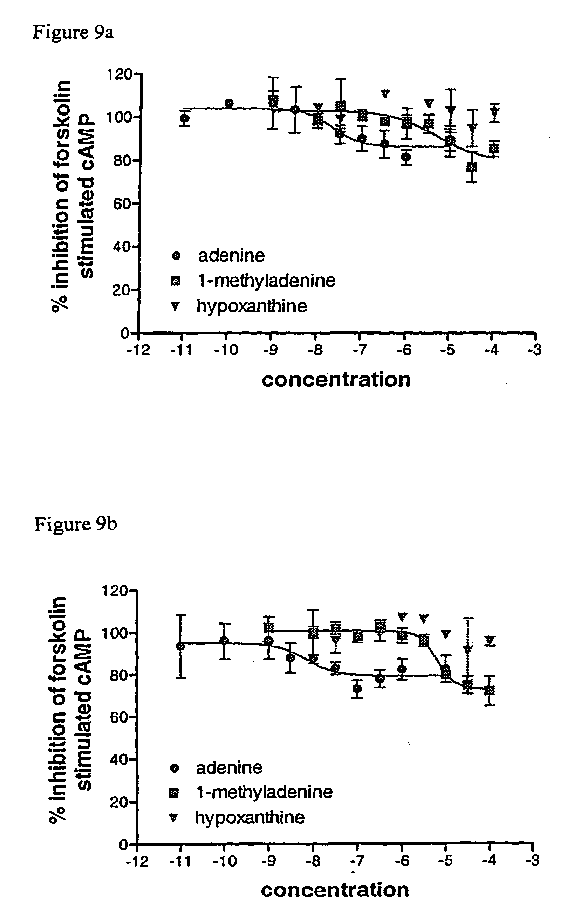

a) Methods - For transiently transfected CHO cells, cAMP measurements were performed using the direct RIA kit from NEN (Belgium).
   The cells were collected from 50 mm petri dishes by adding 1 ml 0.04% EDTA and re-suspending them in PBS (without Ca²⁺ and Mg²⁺). The cells were then centrifuged for 5 minutes at 1,500 RPM, the supernatant was removed and the pellet was re-suspended in stimulation buffer (provided by the supplier, NEN). The cells were counted and 50 µl was added to each well of a 96-well flashplate at a density of 10⁶ cells/well. A standard curve of cAMP was prepared in stimulation buffer, covering a range from 10 to 1,000 nM, and 50 µl was added to the flashplate.
   The compounds were diluted in PBS with forskolin (at 50 µM), and 50 µl of the ligand, 2 times the final concentration, was added to the cells and incubated for 20 minutes at 37 °C. After the 20 minutes incubation, 100 µl of the [¹²⁵I] cAMP tracer (2 µCi) was added to each well, resulting in a final volume of 200 µl/well. The plate was left at room temperature, until being counted in the Topcount^{™} microplate scintillation counter (Packard, Netherlands). The sigmoid concentration response curves (duplicate points) were analyzed using the GraphPad Prism program (GraphPad Software, CA, USA).
b) Results - Inhibition of forskolin stimulated cAMP with the direct method was very low (15-30 %). Forskolin, basal and pEC₅₀ values of both methods are given in table 4; representative curves from the assay are shown in Figure 9a (0.5%hiFCS) and Figure 9b(2%hiFCS).

Although the response window of the assays was low, the assay confirmed the agonistic activity of adenine on the receptor and the measured EC₅₀ (~15 nM) were close to its adenine affinity

Since the low signal in the functional cAMP assay on transiently transfected cells could be due to the heterogeneous nature (transfected and non-transfected cells) of the cells it was decided to select permanently transfected cell lines.

A number of monoclonal cell lines were investigated by radioligand binding as described above with three (clone 26 (mother plate 1), 28, 29 and 30 (mother plate 9) chosen for further studies.

### Example 10 [³⁵S]GTPγS binding in permanently transfected cells

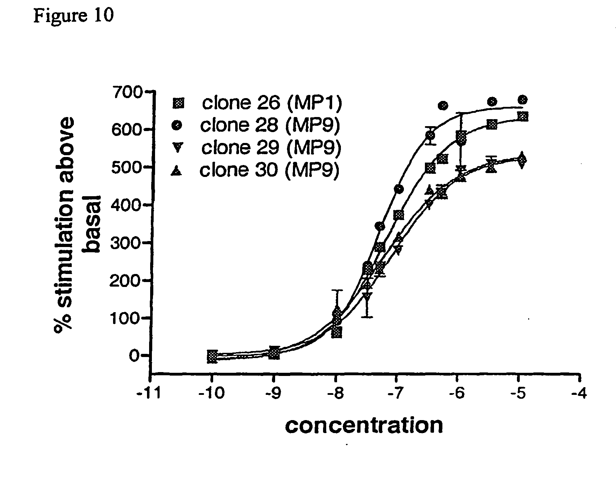

The first functional characterization of the monoclonal cell lines was performed in [³⁵S]GTPγS binding experiments. The maximum stimulation of [³⁵S] GTPγS binding by adenine for each of the 4 selected clones was much higher (500-650-fold) than the that found in the transient transfections confirming the expected better coupling in permanent cell lines (see table 5). The mean EC50 of 62 nM, is however close to the transient preparation (cf Example 8). An example of [³⁵S]GTPγS binding stimulation by adenine is given in Figure 10.

### Example 11 cAMP measurements in permanently transfected CHO cells

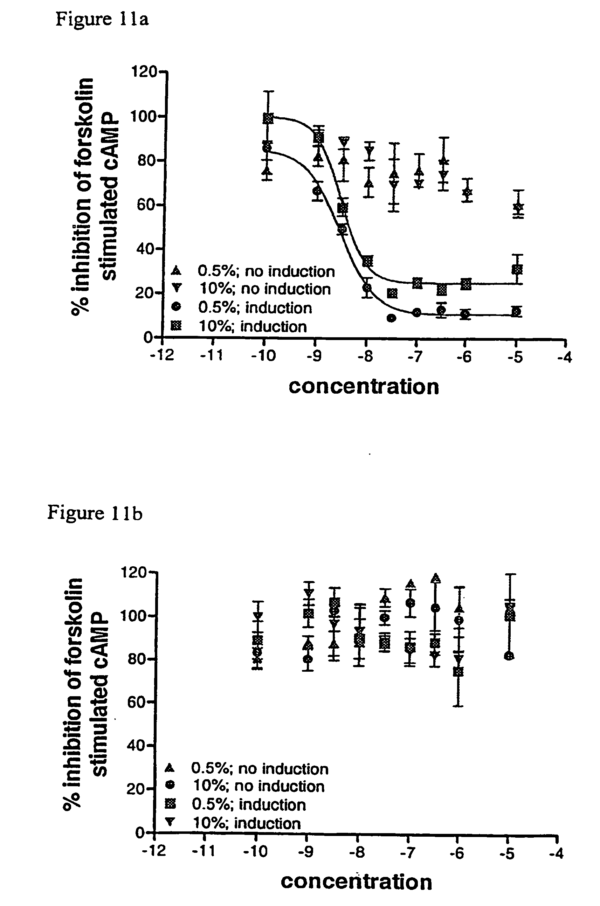

For permanently transfected CHO cells, cAMP measurements were performed using the indirect RIA kit from NEN (Belgium).
a) Methods: Two days before the experiment the cells were seeded out in a 96 well plate at a density required for approximately 90% confluence at the day of the experiment. After removal of the growth medium, the cells were washed twice with a controlled salt solution (CSS) containing 25 mM Tris-HC1, pH 7.4, 120 mM NaCl, 5.4 mM KCl, 0.8 mM MgCl₂, 1.8 mM CaCl₂, 15 mM glucose and 15 mg/l phenol red, with an addition of a phosphodiesterase inhibitor IBMX (3-isobutyl-1-methylxanthine) in a concentration of 1 mM and 0.1% BSA. Basal cAMP levels were determined in CSS containing these additives and maximally stimulated cAMP levels were determined in the presence of 50 µM forskolin. For testing agonistic activity, the compounds were incubated with the cells for 20 minutes at 37 ºC. The reaction was stopped by addition of ice cold HClO₄ (1N) and stored at -20°C. After thawing the mixtures were neutralized with an equivalent amount of phosphate buffered KOH, left at 4ºC during 30 minutes to allow the salts to precipitate and centrifuged at 2,000 RPM for 5 minutes at 4°C.
   For the quantitative determination of the cAMP levels, a 96-well Flashplate radioimmunoassay kit from NEN was used, according to the protocol of the supplier. The sigmoid concentration response curves (duplicate points) were analyzed using the GraphPad Prism program.
b) Results - The effects of Na-butyrate induction and different serum concentrations were evaluated. Investigating the 4 selected clones, clone 28 (MP9) showed highest (-75 %) inhibition of forskolin stimulated cAMP levels. Wild type CHO cells did not show any response to adenine. Forskolin, basal and pEC₅₀ values of clone 28 and wild type CHO cells are given in table 6. A dependable high window in the cAMP experiments was only obtained after Na-butyrate induction of receptor expression levels. The measured EC50 was in the low nanomolar range (3 nM). Representative concentration responses curves are given in Figure 11a and Figure 11b for permanently transfected and wild-type CHO cells respectively.

### Example 12 Intracellular calcium measurements in permanently transfected CHO cells

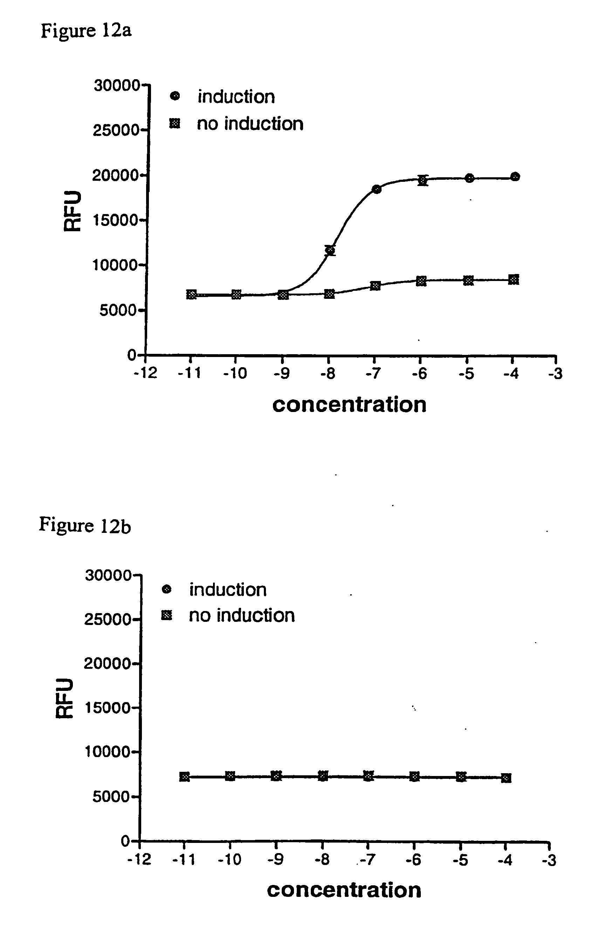

Finally, measurements of intracellular Ca²⁺ were performed on the best functionally coupled CHO cell line, clone 28. The effect of Na-butyrate pre-treatment was investigated using a dose response curve of adenine.
a) Methods - Two days before the experiment the cells were seeded out in a 96 well plate at a density required for approximately 90% cell confluence at the day of the experiment. Compounds were diluted in calcium buffer (5 mM HEPES/NaOH, pH 7.4, 140 mM NaCl, 1 mM MgCl₂, 5 mM KCl, 10 mM glucose, 1.25 mM CaCl₂, 2.5 mM probenecid, pH 7.4), twice their final concentrations. For basal values, calcium buffer was applied and for the maximum response, 10 µM ionomycin was used. The growth medium was removed and the cells were loaded in serum-free growth medium supplemented with probenecid (5 mM), the indicator Fluo-3 (2 µM), 20 mM HEPES/NaOH, pH, 7.4 and 0.1% BSA in the incubator at 37°C. After 60 minutes incubation, the cells were washed 3 times with the calcium buffer. 100 µl of the diluted compounds was transferred to the wells and changes in intracellular calcium levels were measured with the FLIPR^{®} (Fluorometric Imaging Plate Reader, Molecular Devices). Each data point was run in duplicate and the peak of the Ca²⁺-transient was considered as the relevant signal. The sigmoid concentration response curves were analyzed using the GraphPad Prism program.
b) Results - Wild type CHO cells did not show any response to adenine. For non-Na-butyrate treated cells, adenine caused a 1.25-fold increase of the basal relative fluorescence units (RFU) ranging from 6800 to 8500. For the Na-butyrate treated cells, adenine induced a 3-fold increase in RFU ranging from 6600 to 19800. The pEC₅₀ values of the adenine response were 7.14 and 7.83, respectively. A representative example of the Ca²⁺ response triggered by adenine is given in Figure 12a and Figure 12b for permanently transfected and wild-type CHO cells respectively.

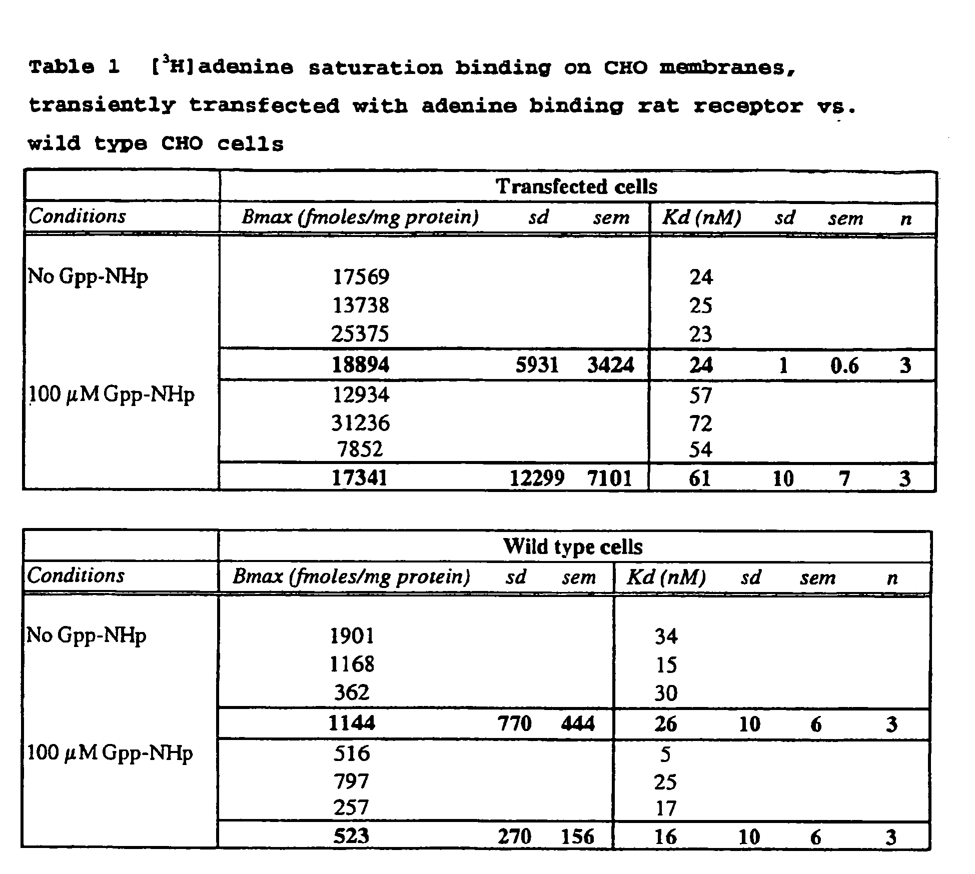

**Table 2: [³H] adenine competition binding on CHO membranes, transiently transfected with adenine binding rat receptor vs. wild type CHO cells**

| | **Transfected cells** | | | | **Wild type cells** | | | |
|---|---|---|---|---|---|---|---|---|
| *Compound listing* | *pIC₅₀* | *sd* | *Kᵢ (nM)* | *n* | *pIC₅₀* | *sd* | *Kᵢ (nM)* | *n* |
| Adenine | 7.51 | | 19 | | 7.13 | | 46 | |
| | 7.48 | | 20 | | 6.8 | | | |
| | 7.76 | | 11 | | 6.67 | | 134 | |
| | 7.44 | | 22 | | | | | |
| | **7.55** | **0.14** | **18** | **4** | **6.87** | **0.24** | **85** | **3** |
| 1-Methyladenine | 5.21 | | 3791 | | < 4 | | | |
| | 5.01 | | 6056 | | < 4 | | | |
| | 5.24 | | 3573 | | | | | |
| | **5.15** | **0.13** | **4391** | **3** | **< 4** | | | **2** |
| AMP | 4.29 | | 31887 | | < 4 | | | |
| | 4.47 | | 20847 | | | | | |
| | 4.46 | | 21210 | | | | | |
| | **4.41** | **0.10** | **24503** | **3** | **< 4** | | | **1** |
| ADP | <4 | | | | < 4 | | | |
| | <4 | | | | | | | |
| | **<4** | | | **2** | **<4** | | | **1** |
| ATP | 4.02 | | 58502 | | < 4 | | | |
| | 4.18 | | 40730 | | | | | |
| | 4.02 | | 58500 | | | | | |
| | **4.07** | **0.09** | **52789** | **3** | **< 4** | | | **1** |
| Adenosine | < 4 | | | | < 4 | | | |
| | 4.14 | | 44554 | | | | | |
| | **4** | | | **2** | **<4** | | | **1** |
| 6-Benzylaminopurine | 4.00 | | 61401 | | < 4 | | | |
| | 4.06 | | 54125 | | | | | |
| | **4.03** | **0.04** | **58328** | **2** | **< 4** | | | **1** |
| Hypoxanthine | <4 | | | | < 4 | | | |
| | <4 | | | | | | | |
| | **<4** | | | **2** | **<4** | | | **1** |
| Guanine | <4 | | | | < 4 | | | |
| | <4 | | | | | | | |
| | **<4** | | | **2** | **<4** | | | **1** |
| Uridine | <4 | | | | < 4 | | | |
| | <4 | | | | | | | |
| | **<4** | | | **2** | **<4** | | | **1** |
| Adrianamycin | 4.35 | | 27792 | | < 4 | | | |
| | **4.35** | | **27918** | **1** | **< 4** | | | **1** |
| Daunomycin | 4.30 | | 30990 | | < 4 | | | |
| | **4.30** | | **31324** | **1** | **<4** | | | **1** |

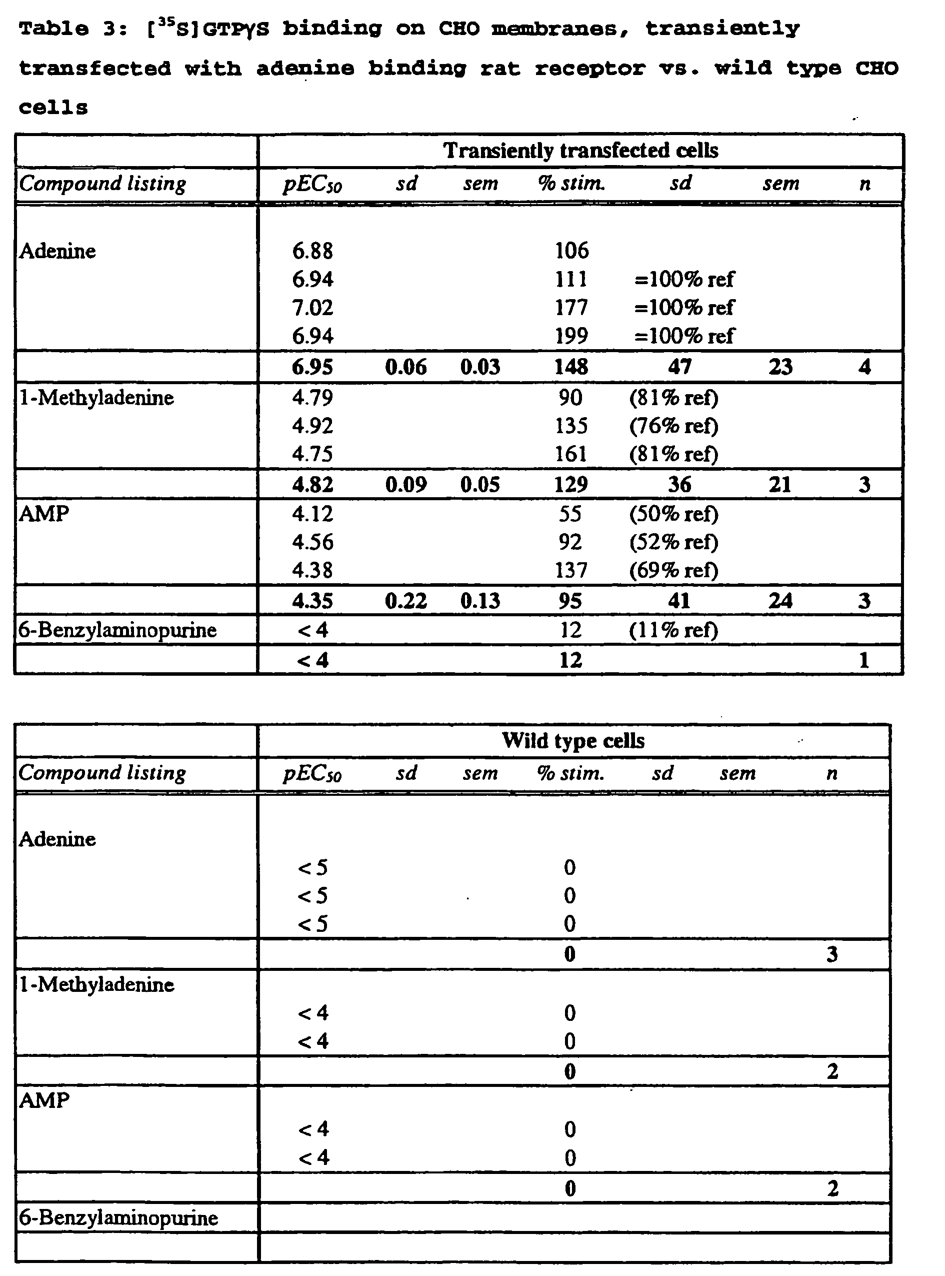

**Table 4: cAMP measurements on CHO cells, transiently transfected with adenine binding rat receptor**

| | | *basal* | *forskolin 50 µM* | *pEC₅₀ adenine* | *pEC*₅₀ *1-methyl adenine* |
|---|---|---|---|---|---|
| | | *(pmol*/*well)* | *(pmol*/*well)* | | |
| Direct assay | | | | | |
| | 0.5%hiFCS | 2.8 | 145 | 7.68 | 5.31 |
| | 2% hiFCS | 2.5 | 105 | 8.15 | 5.20 |
| | | | | | |

| Indirect assay | | | | | |
|---|---|---|---|---|---|
| | 0.5% hiFCS | 0.5 | 74 | 8.48 | 5.10 |
| | 2% hiFCS | 0.4 | 62 | 6.96 | no fit |

**Table 5: [³⁵S]GTPγS binding on CHO membranes, permanently transfected with adenine binding rat receptor. Stimulation above basal by adenine**

| | **Permanently transfected cells** | |
|---|---|---|
| | *pEC₅₀* | *% stimulation* |
| Clone 26 (MP1) | 7.19 | 632 |
| Clone 28 (MP9) | 7.31 | 657 |
| Clone 29 (MP9) | 7.10 | 527 |
| Clone 30 (MP9) | 7.24 | 534 |

**Table 6: cAMP measurements on CHO cells, permanently transfected with adenine binding rat receptor**

| | | *Basal* | *Forskolin 50 µM* | *pEC50 adenine* |
|---|---|---|---|---|
| | | (*pmol*/*well*) | *(pmol*/*well)* | |
| wild type | | | | |
| | 0.5% hiFCS, no induction | 0.27 | 26 | / |
| | 0.5% hiFCS, induction | 0.59 | 25 | / |
| | 10% hiFCS, no induction | 0.64 | 107 | / |
| | 10% hiFCS, induction | 1.08 | 63 | / |
| | | | | |

| transfected cells | | | | |
|---|---|---|---|---|
| | 0.5% hiFCS, no induction | 0.39 | 44 | / |
| | 0.5% hiFCS, induction | 0.71 | 45 | 8.54 |
| | 10% hiFCS, no induction | 0.68 | 142 | / |
| | 10% hiFCS, induction | 1.24 | 65 | 8.53 |

### References:

Altschul,S.F., Madden,T.L., Schaffer,A.A., Zhang, J., Zhang,Z., Miller,W., and Lipman,D.J. (1997) Gapped BLAST and PSI-BLAST: a new generation of protein database search programs. Nucleic Acids Res. 25, 3389-3402.
Birnbaumer, M. (1995) Mutations and diseases of G protein coupled receptors. J. Receptor signal Transduction Res. 15, 131-160.
Buck, L., and Axel, R. (1991) A novel family may encode odorant receptors: a molecular basis for odor recognition. Cell 65, 175-187.
Burnstock, G.(1997) The past, present and future of purine nucleotides as signalling molecules. Neuropharmacology, 36 (9), 1127-1139.
Chow, S.C., Kass, G.E.N., and Orrenius,S., Purines and their roles in apoptosis. Neuropharmacology, 36(9), 1149-1156.
Hinuma, S. et al (1998) A prolactin-releasing peptide in the brain. Nature 393, 272-276.
Libert, F., Parmentier, M., Lefort, A., Dinsart, C., van Sande, J., Maenhaut, Simons, M.-J., Dumont, J.E., and Vassart, G. (1989) Selective amplification and cloning of four new members of the G protein B coupled receptor family. Science 244, 569-572.
Mally, J., and Stone, T.W. (1996) Potential role of adenosine antagonist therapy in pathological tremor disorders. Pharmacol. Ther., 72 (3), 243-250.
Maniatis T., Fritsch E.F., and Sambrook J. (1982) Molecular Cloning: A Laboratory Manual. Cold Spring Harbor, New York.
Meunier J.-C., (1997) Nociceptin/orphanin FQ and the opioid receptor-like ORL1 receptor. Eur. J. Pharmacol. 340, 1-15.
Nyce, J.W. (1999) Insight into adenosine receptor function using antisense and gene-knockout approaches. TiPS 20, 79-83.
Reinscheid R.K. et al., (1995) Orphanin FQ: A neuropeptide that activates an opioidlike G-protein-coupled receptor. Science 270, 792-794.
Sakurai T. et al., (1998) Orexins and orexin receptors: a family of hypothalamic neuropeptide and G-protein-coupled receptors that regulate feeding behaviour. Cell 92, 573-585.
Stadel, J.M., Wilson, S., and Bergsma, D.J. (1997) Orphan G protein-coupled receptors: a neglected opportunity for pioneer drug discovery. TiPS 18, 430-437.

## Claims

1. An isolated nucleic acid sequence encoding an adenine-binding g-protein coupled receptor comprising the polypeptide of SEQ ID NO: 2 or a polypeptide retaining adenine binding receptor activity having at least 70% sequence identity to the polypeptide of SEQ ID NO: 2.

2. The isolated nucleic acid sequence of claim 1, comprising SEQ ID NO: 1 or a sequence having at least 70% sequence identity therewith.

3. An adenine-binding g-protein coupled receptor or a polypeptide retaining adenine binding receptor activity having at least 70% sequence identity to the polypeptide having the sequence of SEQ ID NO: 2.

4. An expression vector comprising a nucleic acid as defined in any one of claims 1 or 2 operably linked to a promoter.

5. An isolated host cell carrying a vector according to claim 4.

6. A nucleic acid primer consisting of a fragment of from 15 to 50 nucleotides of a nucleic acid encoding a polypeptide according to claim 3 that is capable of selectively hybridising to said nucleic acid, optionally linked 5' or 3' to a sequence of 4 to 15 nucleotides to which it is not naturally linked.

7. An assay for a compound capable of interacting with an adenine-binding G protein coupled receptor, which assay comprises:
- providing a G protein coupled receptor comprising at least one polypeptide having adenine-binding activity and encoded by an isolated nucleic acid sequence having at least 70% sequence identity to SEQ ID NO:1;
- contacting said receptor with a putative interacting compound;
- optionally contacting said receptor with adenine; and
- determining whether said compound is an agonist or antagonist of the adenine-induced receptor activation.

8. An assay according to claim 7, wherein said interaction is determined by the ability of the compound to bind to said receptor.

9. An assay according to claim 7, wherein said interaction is determined by the ability of the compound to modulate the activity of said receptor.

10. An assay according to any one of claims 7 to 9, wherein said receptor is located in the membrane of a cell in which the polypeptide according to claim 3 is expressed from a vector according to claim 4.

11. Use of an assay according to any one of claims 7 to 10, for the identification of a modulator compound for the treatment of a disease, the pathology of which is associated with action at purine receptors.

12. The assay of claim 7, wherein said nucleic acid sequence having at least 70% sequence identity to SEQ ID NO:1 is expressed in a cell from a vector comprising said nucleic acid sequence operably linked to a promoter and said assay comprises contacting said cell with said putative interacting compound.

## Patentansprüche

1. Isolierte Nukleinsäuresequenz, codierend für einen Adenin bindenden G-Protein-gekoppelten Rezeptor, der das Polypeptid der SEQ ID NO: 2 umfaßt, oder für ein Polypeptid, bei dem die Adenin bindende Rezeptoraktivität erhalten ist und das eine Sequenzidentität von wenigstens 70% zu dem Polypeptid der SEQ ID NO: 2 aufweist.

2. Isolierte Nukleinsäuresequenz nach Anspruch 1, umfassend die SEQ ID NO: 1 oder eine Sequenz, die eine Sequenzidentität von wenigstens 70% damit aufweist.

3. Adenin bindender G-Protein-gekoppelter Rezeptor oder Polypeptid, bei dem die Adenin bindende Rezeptoraktivität erhalten ist und das eine Sequenzidentität von wenigstens 70% zu dem Polypeptid mit der Sequenz der SEQ ID NO: 2 aufweist.

4. Expressionsvektor, umfassend eine Nukleinsäure mit der in Anspruch 1 oder 2 angegebenen Bedeutung in operativer Verknüpfung mit einem Promotor.

5. Isolierte Wirtszelle mit einem Vektor gemäß Anspruch 4.

6. Nukleinsäureprimer, bestehend aus einem Fragment von 15 bis 50 Nukleotiden einer für ein Polypeptid gemäß Anspruch 3 codierenden Nukleinsäure, der in der Lage ist, selektiv an die Nukleinsäure zu hybridisieren, gegebenenfalls in 5'- oder 3'-Verknüpfung mit einer Sequenz von 4 bis 15 Nukleotiden, mit der er nicht natürlicherweise verknüpft ist.

7. Test für eine Verbindung, die zur Wechselwirkung mit einem Adenin bindenden G-Protein-gekoppelten Rezeptor fähig ist, wobei man bei dem Test
- einen G-Protein-gekoppelten Rezeptor bereitstellt, der wenigstens ein Polypeptid umfaßt, das eine Adenin bindende Aktivität aufweist und von einer isolierten Nukleinsäuresequenz mit einer Sequenzidentität von wenigstens 70% zu SEQ ID NO: 1 codiert wird,
- den Rezeptor mit einer mutmaßlichen wechselwirkenden Verbindung in Kontakt bringt,
- den Rezeptor gegebenenfalls mit Adenin in Kontakt bringt und
- bestimmt, ob es sich bei der Verbindung um einen Agonisten oder Antagonisten der durch Adenin induzierten Rezeptoraktivierung handelt.

8. Test gemäß Anspruch 7, wobei die Wechselwirkung über die Fähigkeit der Verbindung zur Bindung an den Rezeptor bestimmt wird.

9. Test gemäß Anspruch 7, wobei die Wechselwirkung über die Fähigkeit der Verbindung zur Modulation der Aktivität des Rezeptors bestimmt wird.

10. Test gemäß einem der Ansprüche 7 bis 9, wobei der Rezeptor in der Membran einer Zelle, in der das Polypeptid gemäß Anspruch 3 von einem Vektor gemäß Anspruch 4 exprimiert wird, lokalisiert ist.

11. Verwendung eines Tests gemäß einem der Ansprüche 7 bis 10 zur Identifizierung einer Modulatorverbindung zur Behandlung einer Krankheit, deren Krankheitsbild mit einer Wirkung an Purinrezeptoren assoziiert ist.

12. Test nach Anspruch 7, wobei die Nukleinsäuresequenz mit einer Sequenzidentität von wenigstens 70% zu SEQ ID NO: 1 in einer Zelle von einem die Nukleinsäuresequenz in operativer Verknüpfung mit einem Promotor umfassenden Vektor exprimiert wird und man bei dem Test die Zelle mit der mutmaßlichen wechselwirkenden Verbindung in Kontakt bringt.

## Revendications

1. Séquence d'acide nucléique isolée codant pour un récepteur couplé aux protéines G liant l'adénine comprenant le polypeptide de SEQ ID NO : 2 ou un polypeptide conservant l'activité de récepteur liant l'adénine présentant au moins 70% d'identité de séquence avec le polypeptide de SEQ ID NO: 2.

2. Séquence d'acide nucléique isolée selon la revendication 1, comprenant SEQ ID NO: 1 ou une séquence présentant au moins 70% d'identité de séquence avec celle-ci.

3. Récepteur couplé aux protéines G liant l'adénine ou polypeptide conservant l'activité de récepteur liant l'adénine présentant au moins 70% d'identité de séquence avec le polypeptide ayant la séquence de SEQ ID NO: 2.

4. Vecteur d'expression comprenant un acide nucléique tel que défini dans l'une quelconque des revendications 1 ou 2 lié de manière opérationnelle à un promoteur.

5. Cellule hôte isolée portant un vecteur selon la revendication 4.

6. Amorce d'acide nucléique constituée d'un fragment de 15 à 50 nucléotides d'un acide nucléique codant pour un polypeptide selon la revendication 3, qui est susceptible de s'hybrider de façon sélective audit acide nucléique, éventuellement lié en 5' ou en 3' à une séquence de 4 à 15 nucléotides à laquelle il n'est pas naturellement lié.

7. Dosage pour un composé susceptible d'interagir avec un récepteur couplé aux protéines G liant l'adénine, ledit dosage comprenant les étapes consistant à:
- obtenir un récepteur couplé aux protéines G comprenant au moins un polypeptide ayant une activité de liaison à l'adénine et codé par une séquence d'acide nucléique isolée présentant au moins 70 % d'identité de séquence avec SEQ ID NO: 1,
- mettre en contact ledit récepteur avec un composé d'interaction putatif,
- mettre éventuellement en contact ledit récepteur avec de l'adénine; et
- déterminer si ledit composé est un agoniste ou un antagoniste de l'activation du récepteur induite par l'adénine.

8. Dosage selon la revendication 7, selon lequel ladite interaction est déterminée par l'aptitude du composé à se lier audit récepteur.

9. Dosage selon la revendication 7, selon lequel ladite interaction est déterminée par l'aptitude du composé à moduler l'activité dudit récepteur.

10. Dosage selon l'une quelconque des revendications 7 à 9, selon lequel ledit récepteur est situé dans la membrane d'une cellule dans laquelle le polypeptide selon la revendication 3 est exprimé à partir d'un vecteur selon la revendication 4.

11. Utilisation d'un dosage selon l'une quelconque des revendications 7 à 10, pour l'identification d'un composé modulateur pour le traitement d'une maladie dont la pathologie est associée à une action au niveau de récepteurs de purines.

12. Dosage selon la revendication 7, selon lequel ladite séquence d'acide nucléique présentant au moins 70% d'identité de séquence avec SEQ ID NO: 1 est exprimée dans une cellule à partir d'un vecteur comprenant ladite séquence d'acide nucléique liée de manière opérationnelle à un promoteur et ledit dosage comprenant l'étape consistant à mettre en contact ladite cellule avec ledit composé d'interaction putatif.


### SEQUENCE LISTING

<110> Janssen Pharmaceutica N.V.
<120> G Protein Coupled Receptor
<130> Novel GPCR
<140>
   <141>
<150> UK/0008252.9
   <151> 2000-04-04
<160> 9
<170> PatentIn Ver. 2.1
<210> 1
   <211> 999
   <212> DNA
   <213> Rat
<400> 1
   atgggggaaa gcttcaccgg tacagggttt ataaacctaa acacctcagc ctcgacaata 60
   gcagtcacaa caacaaatcc aatggacaaa accatacctg gaagtttcaa cggcaggacc 120
   ctgatcccaa atttgttgat catcatctcg ggactggtcg ggctgatagg aaacgccatg 180
   gtgttctggc tcctgggctt ccgcttagcc aggaatgcct tctcagtcta catcctaaac 240
   ttggccctgg ctgacttcct cttcctcctc tgtcacatca tagattccac gctgctcctt 300
   ctcaagtttt cctaccccaa cattatcttt ctcccatgct ttaacaccgt catgatggtc 360
   ccctacatcg caggcctgag catgctcagt gccatcagca ccgagcgctg cctgtctgtt 420
   gtgtgcccca tctggtatcg ctgccgccgc ccaaaacaca cttcaactgt catgtgctct 480
   gcgatctggg tcctgtcact gttgatctgc attctgaata ggtatttctg tggcttctta 540
   gataccaaat acgaaaagga caaccggtgt ctggcatcga acttctttac tgccgcgtgc 600
   ctgatatttt tgtttgtagt cctctgtctg tccagtctgg ctctgctggt cagatcgttc 660
   tgtggcgctg ggcggatgaa gctcaccaga ttgtacgcga ccattatgct gaccgttttg 720
   gtttttctcc tctgcgggtt gccctttggc atccactggt tcctgttaat ctggattaag 780
   attgattatg gtaaatttgc ttacggtctt tacctggcgg cactcgtcct gactgctgtt 840
   aacagctgtg ccaaccccat catttacttc ttcgtgggct ccttcaggca tcagaagcac 900
   cagaccctca aaatggttct ccagagggcg ctgcaggaca cccctgagac agcggaaaac 960
   acggtggaga tgtcaagcag caaggtggag ccgtgatga                        999
<210> 2
   <211> 331
   <212> PRT
   <213> Rat
<400> 2
   Met Gly Glu Ser Phe Thr Gly Thr Gly Phe Ile Asn Leu Asn Thr Ser
   1 5 10 15
   Ala Ser Thr Ile Ala Val Thr Thr Thr Asn Pro Met Asp Lys Thr Ile
   20 25 30
   Pro Gly Ser Phe Asn Gly Arg Thr Leu Ile Pro Asn Leu Leu Ile Ile
   35 40 45
   Ile Ser Gly Leu Val Gly Leu Ile Gly Asn Ala Met Val Phe Trp Leu
   50 55 60
   Leu Gly Phe Arg Leu Ala Arg Asn Ala Phe Ser Val Tyr Ile Leu Asn
   65 70 75 80
   Leu Ala Leu Ala Asp Phe Leu Phe Leu Leu Cys His Ile Ile Asp Ser
   85 90 95
   Thr Leu Leu Leu Leu Lys Phe Ser Tyr Pro Asn Ile Ile Phe Leu Pro
   100 105 110
   Cys Phe Asn Thr Val Met Met Val Pro Tyr Ile Ala Gly Leu Ser Met
   115 120 125
   Leu Ser Ala Ile Ser Thr Glu Arg Cys Leu Ser Val Val Cys Pro Ile
   130 135 140
   Trp Tyr Arg Cys Arg Arg Pro Lys His Thr Ser Thr Val Met Cys Ser
   145 150 155 160
   Ala Ile Trp Val Leu Ser Leu Leu Ile Cys Ile Leu Asn Arg Tyr Phe
   165 170 175
   Cys Gly Phe Leu Asp Thr Lys Tyr Glu Lys Asp Asn Arg Cys Leu Ala
   180 185 190
   Ser Asn Phe Phe Thr Ala Ala Cys Leu Ile Phe Leu Phe Val Val Leu
   195 200 205
   Cys Leu Ser Ser Leu Ala Leu Leu Val Arg Ser Phe Cys Gly Ala Gly
   210 215 220
   Arg Met Lys Leu Thr Arg Leu Tyr Ala Thr Ile Met Leu Thr Val Leu
   225 230 235 240
   Val Phe Leu Leu Cys Gly Leu Pro Phe Gly Ile His Trp Phe Leu Leu
   245 250 255
   Ile Trp Ile Lys Ile Asp Tyr Gly Lys Phe Ala Tyr Gly Leu Tyr Leu
   260 265 270
   Ala Ala Leu Val Leu Thr Ala Val Asn Ser Cys Ala Asn Pro Ile Ile
   275 280 285
   Tyr Phe Phe Val Gly Ser Phe Arg His Gln Lys His Gln Thr Leu Lys
   290 295 300
   Met Val Leu Gln Arg Ala Leu Gln Asp Thr Pro Glu Thr Ala Glu Asn
   305 310 315 320
   Thr Val Glu Met Ser Ser Ser Lys Val Glu Pro
   325 330
<210> 3
   <211> 969
   <212> DNA
   <213> Human
<400> 3
   atggatccaa ccatctcaac cttggacaca gaactgacac caatcaacgg aactgaggag 60
   actctttgct acaagcagac cttgagcctc acggtgctga cgtgcatcgt ttcccttgtc 120
   gggctgacag gaaacgcagt tgtgctctgg ctcctgggct gccgcatgcg caggaacgcc 180
   ttctccatct acatcctcaa cttggccgca gcagacttcc tcttcctcag cggccgcctt 240
   atatattccc tgttaagctt catcagtatc ccccatacca tctctaaaat cctctatcct 300
   gtgatgatgt ttcctacttt tgcaggcctg agctttctga gtgccgtgag caccgagcgc 360
   tgcctgtccg tcctgtggcc catctggtac cgctgccacc gccccacaca cctgtcagcg 420
   gtggtgtgtg tcctgctctg ggccctgtcc ctgctgcgga gcatcctgga gtggatgtta 480
   tgtggcttcc tgttcagtgg tgctgattct gcttggtgtc aaacatcaga tttcatcaca 540
   gtcgcgtggc tgattttttt atgtgtggtt ctctgtgggt ccagcctggt cctgctgatc 600
   aggattctct gtggatcccg gaagataccg ctgaccaggc tgtacgtgac catcctgctc 660
   acagtactgg tcttcctcct ctgtggcctg ccctttggca ttcagttttt cctattttta 720
   tggatccacg tggacaggga agtcttattt tgtcatgttc atctagtttc tattttcctg 780
   tccgctctta acagcagtgc caaccccatc atttacttct tcgtgggctc ctttaggcag 840
   cgtcaaaata ggcagaacct gaagctggtt ctccagaggg ctctgcagga cgcgtctgag 900
   gtggatgaag gtggagggca gcttcctgag gaaatcctgg agctgtcggg aagcagattg 960
   gagcagtga 969
<210> 4
   <211> 322
   <212> PRT
   <213> Human
<400> 4
   Met Asp Pro Thr Ile Ser Thr Leu Asp Thr Glu Leu Thr Pro Ile Asn
   1 5 10 15
   Gly Thr Glu Glu Thr Leu Cys Tyr Lys Gln Thr Leu Ser Leu Thr Val
   20 25 30
   Leu Thr Cys Ile Val Ser Leu Val Gly Leu Thr Gly Asn Ala Val Val
   35 40 45
   Leu Trp Leu Leu Gly Cys Arg Met Arg Arg Asn Ala Phe Ser Ile Tyr
   50 55 60
   Ile Leu Asn Leu Ala Ala Ala Asp Phe Leu Phe Leu Ser Gly Arg Leu
   65 70 75 80
   Ile Tyr Ser Leu Leu Ser Phe Ile Ser Ile Pro His Thr Ile Ser Lys
   85 90 95
   Ile Leu Tyr Pro Val Met Met Phe Ser Tyr Phe Ala Gly Leu Ser Phe
   100 105 110
   Leu Ser Ala Val Ser Thr Glu Arg Cys Leu Ser Val Leu Trp Pro Ile
   115 120 125
   Trp Tyr Arg Cys His Arg Pro Thr His Leu Ser Ala Val Val Cys Val
   130 135 140
   Leu Leu Trp Ala Leu Ser Leu Leu Arg Ser Ile Leu Glu Trp Met Leu
   145 150 155 160
   Cys Gly Phe Leu Phe Ser Gly Ala Asp Ser Ala Trp Cys Gln Thr Ser
   165 170 175
   Asp Phe Ile Thr Val Ala Trp Leu Ile Phe Leu Cys Val Val Leu Cys
   180 185 190
   Gly Ser Ser Leu Val Leu Leu Ile Arg Ile Leu Cys Gly Ser Arg Lys
   195 200 205
   Ile Pro Leu Thr Arg Leu Tyr Val Thr Ile Leu Leu Thr Val Leu Val
   210 215 220
   Phe Leu Leu Cys Gly Leu Pro Phe Gly Ile Gln Phe Phe Leu Phe Leu
   225 230 235 240
   Trp Ile His Val Asp Arg Glu Val Leu Phe Cys His Val His Leu Val
   245 250 255
   Ser Ile Phe Leu Ser Ala Leu Asn Ser Ser Ala Asn Pro Ile Ile Tyr
   260 265 270
   Phe Phe Val Gly Ser Phe Arg Gln Arg Gln Asn Arg Gln Asn Leu Lys
   275 280 285
   Leu Val Leu Gln Arg Ala Leu Gln Asp Ala Ser Glu Val Asp Glu Gly
   290 295 300
   Gly Gly Gln Leu Pro Glu Glu Ile Leu Glu Leu Ser Gly Ser Arg Leu
   305 310 315 320
   Glu Gln
<210> 5
   <211> 979
   <212> DNA
   <213> Human
<400> 5
   atggccaaat aaaaacagtt ctggtctgca gctcccagcg ggaccaacgc agaagactgg 60
   tgatttctgc atttccaact caggtaccca gttcatctca ttgagactgg ttaggcagtg 120
   tgtacaaccc acggagagca aggagaagca gggtggggca tcgcttcatc tgcgaagtac 180
   aaggagccag gggacacctg tcccccaatc aagggaagct gtgaggaacc ctgctaccag 240
   cccagtacta nnnnnnnnnn nnnnnnnnnn nnnnnnnnnn nnnnnnnnnn nnnnnnnnnn 300
   nnnnnnnnnn nnnnnnnnnn nnnnnnnnnn nnnnnnnnnn nnnnnnnnnn gagtgccatg 360
   agcaccgagc gctgcctgtg cgtcctgtgg cccatatggt accgctgcct cctcccccca 420
   cacacctgtc agcggtcgtg tgtgtcttgc tttgggccct gtccctactg cggagcatcc 480
   tggagtgaat gttctgtgac ttcctgttta gtgatgctga ttctatttgg tgtcaaccat 540
   cagatttcat cacagtcgtg tggctgattt ttttatgtgt ggttctctgt gggtccagcc 600
   tggtcctgct gattaggatt ctctgtggat cctggaagat gcctctgacc gggctgtacg 660
   tgacgatcct gctcacagtg ctagtcttcc tactccgcag cctgcccttc ggcattcggt 720
   gggctctgtc tactgggatc cacctggatt tggaagtcat tttctgtcat gtccatctag 780
   tttccatttt cctgtcccct ctaaacggta gtgccaaccc cgtcatttac ttcttcgtgg 840
   gctcctttag gcagcgtcaa aataggcaga acctgaagct ggttctccag agggctctgc 900
   aggacatgcc tgaggtgaag gtggagggcg gcttcctgag ggaaccctgg agctgtcggg 960
   aagcagattc gggcagtga 979
<210> 6
   <211> 966
   <212> DNA
   <213> Human
<400> 6
   gtggatccaa ccatcccagc cttgggtaca gaactgacac caatcaatgg acgtgaggag 60
   actccttgct acaagcaaac cctgagcctc acagggctga cgtgcatcgt ttcccttgtc 120
   gggatgacag gaaatgcagt cgtgctctgg ctcctgggct tccgcatgcg caggaacgcc 180
   ttctccatct acatcttcaa cctgtccatg gccgacttcc tctttctcag aagccacatt 240
   atacgttttc cgttaagcct catcaatatc ctccatccca tcttcaaaat cctcagccct 300
   gtgatgatgt tttcctacct tgcaagcctg agctttctaa gcgccatgag caccgagcgc 360
   tgcctgtacg tcctgtggcc catctggtag cgctgccgcc cccgccccta cacctgtcag 420
   cggtcgtgtg tgtcatgctc tgggccctgt ctctgctgcg gagcgtcctg gagtggagtt 480
   tctgtgactt cctgtttagt ggtgctgatt ctgtttggtg ttaaacatca gatttcatca 540
   tagtaggggg gctgattttt ttatgtgtgg ctctctgtgg ttccagcctg gtcctgctgg 600
   tcaggatcct ttgtgggtcc cggaagatgc cactgaccag gctgtacgtg accatcctgc 660
   tcatagcgct ggtcttcctc ctctgtggcc tgccctttgg cattcggttt ttcctatttt 720
   catggaacca cgtggatttg gaagtcttat attgtcacgt tcatctagtt tccattttcc 780
   tttcctctct taacggccaa ccccaacatt tacttcttcg tgggctcctt taggcagtgt 840
   caaaataggc agaacctgaa gctggttctc cagagggctc tgcaggacac gactgaggtg 900
   aatgaaggtg gacgatggct tcctgaggaa accctggagc tgtcaggaag cagatttggg 960
   cagtga                                                            966
<210> 7
   <211> 961
   <212> DNA
   <213> Human
<400> 7
   atggatccaa ccatcccagc cttgggtaca gaactgacat caatcaatag gaccgaggag 60
   acccatcttc aacattgtgg catggagatc atgatcctca tgttgctgct cctcatcgtt 120
   gacctggtcc agctggcagg aaatgcagtc atttcctctg gctcctggga ttccgcctgc 180
   acaggaacac cttctccctc tacaccctca acctggccgg ggccgacttc ttcctctgct 240
   cccagatttt agaaattgtg aatttctacc atgacttctt cctttccatc tccacctatt 300
   tcaccactgt gatgaccttt ctctacttta caggcctgag catgctgggc tccatcagca 360
   ccaagcactg cctgtccatc ctgtggccca tctagtaccg ctgccaccac cccacacacc 420
   tgtcagcagt cgtgtgtcct gctctgggcc ctgtccctgc tgcagagcat cctggaatgg 480
   atgttctgtg gcttcctgtc tagtggtgct gattctgttt ggtgtgaaac atcagatttc 540
   atcacagtca catggctgat ttttttatgt gtggttctct gcgggtccag cccggtcctg 600
   ctggtcagga tcctttgtgg atcccggaag atgcccttga ccaggctgta catgaccatc 660
   ctgctcagag tgctggtctt cctcctctgt gacctgccct ttggcattca gtgattccta 720
   tttttctgga tccacgtgga tttgtcacgt tcgtctagtt tccattttcc tgtccactct 780
   taacagcagt gccaacccca ttatttactt cttcatgggc tcctttaggc agcttcaaaa 840
   caggaagact ctctagctgg ttctccagag ggctctgcag gacacgcctg aggtggaaga 900
   aggcagatgg cggctttctg aggaaaccct ggagctgtca tgaagcagat tggggccatg 960
   a                                                                 961
<210> 8
   <211> 994
   <212> DNA
   <213> Human
<400> 8
   atgaatccaa ccatcccagc cttggataca gaaattgcac caattagtga tacagaggag 60
   acccatcctc atcgttgtgg catggaggtc ctggtcctca tagtgctgat cctcatcatt 120
   gacctggtcg ggctggcagg aaatgcagtc atgctctggc tcctgggctt ctgcatgcac 180
   agtaacacct tctctctcta catcctcaac ctggccaggg ctgacttcct ctgcacctgc 240
   ttccagatta taacattcat taatttcttc agtgactttg ttagttctct ctccatccat 300
   ttctctagat ttgtcaccac ggtgttgttc tccgcctgta ttacaggcct gagcatgctg 360
   agcaccatca gcaccgagca ccgcctgtcc gtcctgtggc ccatctggta ctgctgccac 420
   tgccccacac acctgtcagc ggtcatgtgt gtcctgctct gggccctgtc cctgttgcag 480
   agcatcctgg agtggatgtt ctgtagcttc ctgtttagtg atgttgactc tgataattgg 540
   tgtcaaatat tagatttcct cactgctgtg tggctgattt ttttaatctg tggttctctg 600
   tgggttcacc ctggtcctgc ttgtcaggat catatgtgga tcccagaaga tgccgctgac 660
   caggctgtat gtgaccatcc tgctcacagg gctggtcttc ctcttctgca gcctgcccct 720
   cagcattcag tgattcctat tatactggat cgagaaggat ttggatgact taccttgtgt 780
   tgttcgttta atttccattt tcctgtctgc tcttaacagc agtgccaacc ccatcattta 840
   cttcttcatg ggctccttta ggcagcttca aaacaggaag accctcaagc tggttctcca 900
   gagggctctg caggacatgc ttgaggtgga tgaaggtgga gggcagcttc ctgaggaaac 960
   cctgaagctg tcgggaagca gattggggcc atga 994
<210> 9
   <211> 966
   <212> DNA
   <213> Human
<400> 9
   atggatccaa ccatcccagt cttgggtaca gaactgacac caatcaacgg aactgaggag 60
   actccttgct acaatcagac cctgagcttc acggtgctga cgtgcatcgt ttcccttgtc 120
   gcgctgacag gaaacgcggt tgtgctctgg ctcctgggct tccgcatgtg caggaacgct 180
   gtctccatct acatcctcaa cctggtcgcg gccaacttcc tcctcctcag cagccacatt 240
   atacatccct gttacacctc atcaataacg tccatcccat ctctctaatc ctctagcctg 300
   tgatgacctt tccctacttg gcaggcctga atattctgag tgccatgagc accaagcgct 360
   gcctgtcaat cctgtggccc atctggtaac gctgccgcca ccccacacac ctgtcaacgg 420
   tcgtgtgtgt cctgctctgg gccctgtccc tgctgtagag catcctggag tggatgttct 480
   gtgactccct gtttagtgat gctgattctg tttggtgtca aacatcagat tcatcacagt 540
   tacgtggctg atttttttat ttgtggttct ctgtgtgtcc agcctggtcc tagtggtcag 600
   gatcctctgt ggatcccaga agatgccgct gaccaggctg tacatgacca tctgctcaca 660
   gtgctggtct tcctcctctg cggcctgccc attggcattc agtgggccct gttttccagg 720
   atccacatgg actgggaagt cttatattct catgttcatc tgccttccat tttcctgtcg 780
   tctcttaaca gcagtgccaa ccccatcatt tacttcttca tgggtttcgt taggcagcat 840
   caaaattggc agaacctgaa gctggttctc cagagggatc tgcaggacac gcctgaggtg 900
   gatgaaggtg gatggtgcct tcctcaggaa accctggagc tgtcgggaag cagattcagg 960
   cagtga 966

### SEQUENCE LISTING

<110> Janssen Pharmaceutica N.V.
<120> G Protein Coupled Receptor
<130> Novel GPCR
<140>
   <141>
<150> UK/0008252.9
   <151> 2000-04-04
<160> 9
<170> PatentIn Ver. 2.1
<210> 1
   <211> 999
   <212> DNA
   <213> Rattus norvegicus
<400> 1
   atgggggaaa gcttcaccgg tacagggttt ataaacctaa acacctcagc ctcgacaata 60
   gcagtcacaa caacaaatcc aatggacaaa accatacctg gaagtttcaa cggcaggacc 120
   ctgatcccaa atttgttgat catcatctcg ggactggtcg ggctgatagg aaacgccatg 180
   gtgttctggc tcctgggctt ccgcttagcc aggaatgcct tctcagtcta catcctaaac 240
   ttggccctgg ctgacttcct cttcctcctc tgtcacatca tagattccac gctgctcctt 300
   ctcaagtttt cctaccccaa cattatcttt ctcccatgct ttaacaccgt catgatggtc 360
   ccctacatcg caggcctgag catgctcagt gccatcagca ccgagcgctg cctgtctgtt 420
   gtgtgcccca tctggtatcg ctgccgccgc ccaaaacaca cttcaactgt catgtgctct 480
   gcgatctggg tcctgtcact gttgatctgc attctgaata ggtatttctg tggcttctta 540
   gataccaaat acgaaaagga caaccggtgt ctggcatcga acttctttac tgccgcgtgc 600
   ctgatatttt tgtttgtagt cctctgtctg tccagtctgg ctctgctggt cagatcgttc 660
   tgtggcgctg ggcggatgaa gctcaccaga ttgtacgcga ccattatgct gaccgttttg 720
   gtttttctcc tctgcgggtt gccctttggc atccactggt tcctgttaat ctggattaag 780
   attgattatg gtaaatttgc ttacggtctt tacctggcgg cactcgtcct gactgctgtt 840
   aacagctgtg ccaaccccat catttacttc ttcgtgggct ccttcaggca tcagaagcac 900
   cagaccctca aaatggttct ccagagggcg ctgcaggaca cccctgagac agcggaaaac 960
   acggtggaga tgtcaagcag caaggtggag ccgtgatga                        999
<210> 2
   <211> 331
   <212> PRT
   <213> Rattus norvegicus
<400> 2
   Met Gly Glu Ser Phe Thr Gly Thr Gly Phe Ile Asn Leu Asn Thr Ser
    1               5                  10                  15
   Ala Ser Thr Ile Ala Val Thr Thr Thr Asn Pro Met Asp Lys Thr Ile
               20                  25                  30
   Pro Gly Ser Phe Asn Gly Arg Thr Leu Ile Pro Asn Leu Leu Ile Ile
           35                  40                  45
   Ile Ser Gly Leu Val Gly Leu Ile Gly Asn Ala Met Val Phe Trp Leu
       50                  55                  60
   Leu Gly Phe Arg Leu Ala Arg Asn Ala Phe Ser Val Tyr Ile Leu Asn
   65                  70                  75                  80
   Leu Ala Leu Ala Asp Phe Leu Phe Leu Leu Cys His Ile Ile Asp Ser
                   85                  90                  95
   Thr Leu Leu Leu Leu Lys Phe Ser Tyr Pro Asn Ile Ile Phe Leu Pro
               100                 105                 110
   Cys Phe Asn Thr Val Met Met Val Pro Tyr Ile Ala Gly Leu Ser Met
           115                 120                 125
   Leu Ser Ala Ile Ser Thr Glu Arg Cys Leu Ser Val Val Cys Pro Ile
       130                 135                 140
   Trp Tyr Arg Cys Arg Arg Pro Lys His Thr Ser Thr Val Met Cys Ser
   145                 150                 155                 160
   Ala Ile Trp Val Leu Ser Leu Leu Ile Cys Ile Leu Asn Arg Tyr Phe
                   165                 170                 175
   Cys Gly Phe Leu Asp Thr Lys Tyr Glu Lys Asp Asn Arg Cys Leu Ala
               180                 185                 190
   Ser Asn Phe Phe Thr Ala Ala Cys Leu Ile Phe Leu Phe Val Val Leu
           195                 200                 205
   Cys Leu Ser Ser Leu Ala Leu Leu Val Arg Ser Phe Cys Gly Ala Gly
       210                 215                 220
   Arg Met Lys Leu Thr Arg Leu Tyr Ala Thr Ile Met Leu Thr Val Leu
   225                 230                 235                 240
   Val Phe Leu Leu Cys Gly Leu Pro Phe Gly Ile His Trp Phe Leu Leu
                   245                 250                 255
   Ile Trp Ile Lys Ile Asp Tyr Gly Lys Phe Ala Tyr Gly Leu Tyr Leu
   260 265 270
   Ala Ala Leu Val Leu Thr Ala Val Asn Ser Cys Ala Asn Pro Ile Ile
   275 280 285
   Tyr Phe Phe Val Gly Ser Phe Arg His Gln Lys His Gln Thr Leu Lys
   290 295 300
   Met Val Leu Gln Arg Ala Leu Gln Asp Thr Pro Glu Thr Ala Glu Asn
   305 310 315 320
   Thr Val Glu Met Ser Ser Ser Lys Val Glu Pro
   325 330
<210> 3
   <211> 969
   <212> DNA
   <213> Homo sapiens
<400> 3
   atggatccaa ccatctcaac cttggacaca gaactgacac caatcaacgg aactgaggag 60
   actctttgct acaagcagac cttgagcctc acggtgctga cgtgcatcgt ttcccttgtc 120
   gggctgacag gaaacgcagt tgtgctctgg ctcctgggct gccgcatgcg caggaacgcc 180
   ttctccatct acatcctcaa cttggccgca gcagacttcc tcttcctcag cggccgcctt 240
   atatattccc tgttaagctt catcagtatc ccccatacca tctctaaaat cctctatcct 300
   gtgatgatgt tttcctactt tgcaggcctg agctttctga gtgccgtgag caccgagcgc 360
   tgcctgtccg tcctgtggcc catctggtac cgctgccacc gccccacaca cctgtcagcg 420
   gtggtgtgtg tcctgctctg ggccctgtcc ctgctgcgga gcatcctgga gtggatgtta 480
   tgtggcttcc tgttcagtgg tgctgattct gcttggtgtc aaacatcaga tttcatcaca 540
   gtcgcgtggc tgattttttt atgtgtggtt ctctgtgggt ccagcctggt cctgctgatc 600
   aggattctct gtggatcccg gaagataccg ctgaccaggc tgtacgtgac catcctgctc 660
   acagtactgg tcttcctcct ctgtggcctg ccctttggca ttcagttttt cctattttta 720
   tggatccacg tggacaggga agtcttattt tgtcatgttc atctagtttc tattttcctg 780
   tccgctctta acagcagtgc caaccccatc atttacttct tcgtgggctc ctttaggcag 840
   cgtcaaaata ggcagaacct gaagctggtt ctccagaggg ctctgcagga cgcgtctgag 900
   gtggatgaag gtggagggca gcttcctgag gaaatcctgg agctgtcggg aagcagattg 960
   gagcagtga                                                         969
<210> 4
   <211> 322
   <212> PRT
   <213> Homo sapiens
<400> 4
   Met Asp Pro Thr Ile Ser Thr Leu Asp Thr Glu Leu Thr Pro Ile Asn
   1 5 10 15
   Gly Thr Glu Glu Thr Leu Cys Tyr Lys Gln Thr Leu Ser Leu Thr Val
   20 25 30
   Leu Thr Cys Ile Val Ser Leu Val Gly Leu Thr Gly Asn Ala Val Val
   35 40 45
   Leu Trp Leu Leu Gly Cys Arg Met Arg Arg Asn Ala Phe Ser Ile Tyr
   50 55 60
   Ile Leu Asn Leu Ala Ala Ala Asp Phe Leu Phe Leu Ser Gly Arg Leu
   65 70 75 80
   Ile Tyr Ser Leu Leu Ser Phe Ile Ser Ile Pro His Thr Ile Ser Lys
   85 90 95
   Ile Leu Tyr Pro Val Met Met Phe Ser Tyr Phe Ala Gly Leu Ser Phe
   100 105 110
   Leu Ser Ala Val Ser Thr Glu Arg Cys Leu Ser Val Leu Trp Pro Ile
   115 120 125
   Trp Tyr Arg Cys His Arg Pro Thr His Leu Ser Ala Val Val Cys Val
   130 135 140
   Leu Leu Trp Ala Leu Ser Leu Leu Arg Ser Ile Leu Glu Trp Met Leu
   145 150 155 160
   Cys Gly Phe Leu Phe Ser Gly Ala Asp Ser Ala Trp Cys Gln Thr Ser
   165 170 175
   Asp Phe Ile Thr Val Ala Trp Leu Ile Phe Leu Cys Val Val Leu Cys
   180 185 190
   Gly Ser Ser Leu Val Leu Leu Ile Arg Ile Leu Cys Gly Ser Arg Lys
   195 200 205
   Ile Pro Leu Thr Arg Leu Tyr Val Thr Ile Leu Leu Thr Val Leu Val
   210 215 220
   Phe Leu Leu Cys Gly Leu Pro Phe Gly Ile Gln Phe Phe Leu Phe Leu
   225 230 235 240
   Trp Ile His Val Asp Arg Glu Val Leu Phe Cys His Val His Leu Val
   245 250 255
   Ser Ile Phe Leu Ser Ala Leu Asn Ser Ser Ala Asn Pro Ile Ile Tyr
   260 265 270
   Phe Phe Val Gly Ser Phe Arg Gln Arg Gln Asn Arg Gln Asn Leu Lys
   275 280 285
   Leu Val Leu Gln Arg Ala Leu Gln Asp Ala Ser Glu Val Asp Glu Gly
   290 295 300
   Gly Gly Gln Leu Pro Glu Glu Ile Leu Glu Leu Ser Gly Ser Arg Leu
   305 310 315 320
   Glu Gln
<210> 5
   <211> 979
   <212> DNA
   <213> Homo sapiens
<220>
   <221> misc_feature
   <222> (251)..(350)
   <223> n=a, t, c or g
<400> 5
   atggccaaat aaaaacagtt ctggtctgca gctcccagcg ggaccaacgc agaagactgg 60
   tgatttctgc atttccaact caggtaccca gttcatctca ttgagactgg ttaggcagtg 120
   tgtacaaccc acggagagca aggagaagca gggtggggca tcgcttcatc tgcgaagtac 180
   aaggagccag gggacacctg tcccccaatc aagggaagct gtgaggaacc ctgctaccag 240
   cccagtacta nnnnnnnnnn nnnnnnnnnn nnnnnnnnnn nnnnnnnnnn nnnnnnnnnn 300
   nnnnnnnnnn nnnnnnnnnn nnnnnnnnnn nnnnnnnnnn nnnnnnnnnn gagtgccatg 360
   agcaccgagc gctgcctgtg cgtcctgtgg cccatatggt accgctgcct cctcccccca 420
   cacacctgtc agcggtcgtg tgtgtcttgc tttgggccct gtccctactg cggagcatcc 480
   tggagtgaat gttctgtgac ttcctgttta gtgatgctga ttctatttgg tgtcaaccat 540
   cagatttcat cacagtcgtg tggctgattt ttttatgtgt ggttctctgt gggtccagcc 600
   tggtcctgct gattaggatt ctctgtggat cctggaagat gcctctgacc gggctgtacg 660
   tgacgatcct gctcacagtg ctagtcttcc tactccgcag cctgcccttc ggcattcggt 720
   gggctctgtc tactgggatc cacctggatt tggaagtcat tttctgtcat gtccatctag 780
   tttccatttt cctgtcccct ctaaacggta gtgccaaccc cgtcatttac ttcttcgtgg 840
   gctcctttag gcagcgtcaa aataggcaga acctgaagct ggttctccag agggctctgc 900
   aggacatgcc tgaggtgaag gtggagggcg gcttcctgag ggaaccctgg agctgtcggg 960
   aagcagattc gggcagtga 979
<210> 6
   <211> 966
   <212> DNA
   <213> Homo sapiens
<400> 6
   gtggatccaa ccatcccagc cttgggtaca gaactgacac caatcaatgg acgtgaggag 60
   actccttgct acaagcaaac cctgagcctc acagggctga cgtgcatcgt ttcccttgtc 120
   gggatgacag gaaatgcagt cgtgctctgg ctcctgggct tccgcatgcg caggaacgcc 180
   ttctccatct acatcttcaa cctgtccatg gccgacttcc tctttctcag aagccacatt 240
   atacgttttc cgttaagcct catcaatatc ctccatccca tcttcaaaat cctcagccct 300
   gtgatgatgt tttcctacct tgcaagcctg agctttctaa gcgccatgag caccgagcgc 360
   tgcctgtacg tcctgtggcc catctggtag cgctgccgcc cccgccccta cacctgtcag 420
   cggtcgtgtg tgtcatgctc tgggccctgt ctctgctgcg gagcgtcctg gagtggagtt 480
   tctgtgactt cctgtttagt ggtgctgatt ctgtttggtg ttaaacatca gatttcatca 540
   tagtaggggg gctgattttt ttatgtgtgg ctctctgtgg ttccagcctg gtcctgctgg 600
   tcaggatcct ttgtgggtcc cggaagatgc cactgaccag gctgtacgtg accatcctgc 660
   tcatagcgct ggtcttcctc ctctgtggcc tgccctttgg cattcggttt ttcctatttt 720
   catggaacca cgtggatttg gaagtcttat attgtcacgt tcatctagtt tccattttcc 780
   tttcctctct taacggccaa ccccaacatt tacttcttcg tgggctcctt taggcagtgt 840
   caaaataggc agaacctgaa gctggttctc cagagggctc tgcaggacac gactgaggtg 900
   aatgaaggtg gacgatggct tcctgaggaa accctggagc tgtcaggaag cagatttggg 960
   cagtga                                                            966
<210> 7
   <211> 961
   <212> DNA
   <213> Homo sapiens
<400> 7
   atggatccaa ccatcccagc cttgggtaca gaactgacat caatcaatag gaccgaggag 60
   acccatcttc aacattgtgg catggagatc atgatcctca tgttgctgct cctcatcgtt 120
   gacctggtcc agctggcagg aaatgcagtc atttcctctg gctcctggga ttccgcctgc 180
   acaggaacac cttctccctc tacaccctca acctggccgg ggccgacttc ttcctctgct 240
   cccagatttt agaaattgtg aatttctacc atgacttctt cctttccatc tccacctatt 300
   tcaccactgt gatgaccttt ctctacttta caggcctgag catgctgggc tccatcagca 360
   ccaagcactg cctgtccatc ctgtggccca tctagtaccg ctgccaccac cccacacacc 420
   tgtcagcagt cgtgtgtcct gctctgggcc ctgtccctgc tgcagagcat cctggaatgg 480
   atgttctgtg gcttcctgtc tagtggtgct gattctgttt ggtgtgaaac atcagatttc 540
   atcacagtca catggctgat ttttttatgt gtggttctct gcgggtccag cccggtcctg 600
   ctggtcagga tcctttgtgg atcccggaag atgcccttga ccaggctgta catgaccatc 660
   ctgctcagag tgctggtctt cctcctctgt gacctgccct ttggcattca gtgattccta 720
   tttttctgga tccacgtgga tttgtcacgt tcgtctagtt tccattttcc tgtccactct 780
   taacagcagt gccaacccca ttatttactt cttcatgggc tcctttaggc agcttcaaaa 840
   caggaagact ctctagctgg ttctccagag ggctctgcag gacacgcctg aggtggaaga 900
   aggcagatgg cggctttctg aggaaaccct ggagctgtca tgaagcagat tggggccatg 960
   a                                                                 961
<210> 8
   <211> 994
   <212> DNA
   <213> Homo sapiens
<400> 8
   atgaatccaa ccatcccagc cttggataca gaaattgcac caattagtga tacagaggag 60
   acccatcctc atcgttgtgg catggaggtc ctggtcctca tagtgctgat cctcatcatt 120
   gacctggtcg ggctggcagg aaatgcagtc atgctctggc tcctgggctt ctgcatgcac 180
   agtaacacct tctctctcta catcctcaac ctggccaggg ctgacttcct ctgcacctgc 240
   ttccagatta taacattcat taatttcttc agtgactttg ttagttctct ctccatccat 300
   ttctctagat ttgtcaccac ggtgttgttc tccgcctgta ttacaggcct gagcatgctg 360
   agcaccatca gcaccgagca ccgcctgtcc gtcctgtggc ccatctggta ctgctgccac 420
   tgccccacac acctgtcagc ggtcatgtgt gtcctgctct gggccctgtc cctgttgcag 480
   agcatcctgg agtggatgtt ctgtagcttc ctgtttagtg atgttgactc tgataattgg 540
   tgtcaaatat tagatttcct cactgctgtg tggctgattt ttttaatctg tggttctctg 600
   tgggttcacc ctggtcctgc ttgtcaggat catatgtgga tcccagaaga tgccgctgac 660
   caggctgtat gtgaccatcc tgctcacagg gctggtcttc ctcttctgca gcctgcccct 720
   cagcattcag tgattcctat tatactggat cgagaaggat ttggatgact taccttgtgt 780
   tgttcgttta atttccattt tcctgtctgc tcttaacagc agtgccaacc ccatcattta 840
   cttcttcatg ggctccttta ggcagcttca aaacaggaag accctcaagc tggttctcca 900
   gagggctctg caggacatgc ttgaggtgga tgaaggtgga gggcagcttc ctgaggaaac 960
   cctgaagctg tcgggaagca gattggggcc atga                            994
<210> 9
   <211> 966
   <212> DNA
   <213> Homo sapiens
<400> 9
   atggatccaa ccatcccagt cttgggtaca gaactgacac caatcaacgg aactgaggag 60
   actccttgct acaatcagac cctgagcttc acggtgctga cgtgcatcgt ttcccttgtc 120
   gcgctgacag gaaacgcggt tgtgctctgg ctcctgggct tccgcatgtg caggaacgct 180
   gtctccatct acatcctcaa cctggtcgcg gccaacttcc tcctcctcag cagccacatt 240
   atacatccct gttacacctc atcaataacg tccatcccat ctctctaatc ctctagcctg 300
   tgatgacctt tccctacttg gcaggcctga atattctgag tgccatgagc accaagcgct 360
   gcctgtcaat cctgtggccc atctggtaac gctgccgcca ccccacacac ctgtcaacgg 420
   tcgtgtgtgt cctgctctgg gccctgtccc tgctgtagag catcctggag tggatgttct 480
   gtgactccct gtttagtgat gctgattctg tttggtgtca aacatcagat tcatcacagt 540
   tacgtggctg atttttttat ttgtggttct ctgtgtgtcc agcctggtcc tagtggtcag 600
   gatcctctgt ggatcccaga agatgccgct gaccaggctg tacatgacca tctgctcaca 660
   gtgctggtct tcctcctctg cggcctgccc attggcattc agtgggccct gttttccagg 720
   atccacatgg actgggaagt cttatattct catgttcatc tgccttccat tttcctgtcg 780
   tctcttaaca gcagtgccaa ccccatcatt tacttcttca tgggtttcgt taggcagcat 840
   caaaattggc agaacctgaa gctggttctc cagagggatc tgcaggacac gcctgaggtg 900
   gatgaaggtg gatggtgcct tcctcaggaa accctggagc tgtcgggaag cagattcagg 960
   cagtga                                                           966